# EUROPEAN PATENT APPLICATION

(11) **EP 4 843 525 A1**
(43) Date of publication of application: **25.11.2026**
(21) Application number: 25177976.5
(22) Date of filing: 21.05.2025
(51) Int. Cl.: A61B 18/20

(54) **APPARATUS AND METHOD FOR LASER TREATMENT OF A TISSUE SURFACE**

(71) Applicant: Fotona d.o.o., 1000 Ljubljana (SI)
(72) Inventor: LUKAC, Matjaz, 1000 Ljubljana (SI); KOREN, Monika, 4240 Radovljica (SI); KOSIR, Jure, 1230 Domzale (SI); SENEGACNIK, Matej, 1000 Ljubljana (SI); ZEL, Tilen, 2234 Benedikt (SI)
(74) Representative: Bardehle Pagenberg Partnerschaft mbB Patentanwälte Rechtsanwälte

(57) **Abstract**

An apparatus for repetitively emitting trains of laser pulses (100) onto an organic tissue is disclosed. Each train of laser pulses includes at least one laser pulse (110) of a first type and at least one laser pulse (120) of a second type. A time d between a last one of the at least one laser pulse of the first type and a first one of the at least one laser pulse of the second type is no more than 500 ms, preferably shorter than 300 ms, more preferably shorter than 100 ms. The at least one laser pulse of the first type comprises a fluence below an ablation threshold of the organic tissue, preferably at least 10% below the ablation threshold. The at least one laser pulse of the second type comprises a fluence above the ablation threshold of the organic tissue, preferably at least 10% above the ablation threshold. The train of laser pulses starts with a pulse of the first type and/or ends with a pulse of the second type.

## Description

### 1. Field of the invention

The present invention relates to an apparatus and a method for laser treatment of an organic tissue including different pulse types, such as including different durations, powers and/or wavelengths, as well as corresponding computer programs.

### 2. Technical background

Lasers are used for treating a wide variety of medical and cosmetic conditions. For efficacy and safety reasons the laser parameters must be carefully controlled and selected for each particular condition. Such parameters may include laser wavelength and laser pulse shape, duration, energy and repetition rate.

For successful operation of such lasers, energy needs to be delivered to the active medium of the laser, that determines the laser's output wavelength, via a pumping mechanism that may include, e.g., diode pumping, optical pumping or electrical pumping. To deliver energy to the active medium, specialized circuits are needed so that power is precisely controlled to limit output laser parameter deviations with potentially harmful effects to the patient.

A pulse forming module (PFM) of a power supply of a laser usually consists of a large storage capacitor or multiple storage capacitors connected in series and/or in parallel. The storage capacitor(s) is/are typically charged to a voltage between 100 and 1500 Volts (V) and comprise(s) a (total) capacitance in the range from 100 microfarad to tens of millifarad. Energy is transferred from the storage capacitor(s) to the pumping mechanism, i.e., a pump such as a flashlight, and in turn to the active medium of the laser by means of an electronic switch causing the storage capacitor(s) to be discharged into the pump, which, e.g., in the case of a pump in the shape of a flashlight produces intense light which is used to pump the active medium. As the storage capacitor(s) can be neither quickly charged nor discharged without discharging through the pump, it is typically not possible to significantly change laser pulse parameters from pulse to pulse when pulses follow each other with a very short delay. Therefore, different conditions are typically treated separately, with individual treatment steps performed with a substantial time gap in-between them.

The above separate treatment step approach is typically applied also when using different laser wavelengths for treating different conditions. This is because human tissues are multi-component composite systems, with generally very complex interactions with laser light.

Within the UVB (280-320 nm), UVA (320-400 nm) and visible light (400-700 nm) spectral regions, skin has three primary absorbers: blood (hemoglobin), melanosomes (melanin), and keratin. Despite its abundance in all tissues, water is not a significant absorber of light in the visible region. However, above approximately 1000 nm it becomes a primary absorber, considerably simplifying the interaction mechanism.

Among mid-infrared lasers, the laser wavelengths most suitable for water-mediated treatments are located at the two highest water absorption peaks, corresponding to a first range from 2.5 µm to 3.5 µm, preferably from 2.7 µm to 3 µm, and to a second range from 8 µm to 11 µm, preferably from 9 µm to 11 µm. For laser wavelengths within these two ranges the optical absorption depth within the tissue is less than about 100 µm. Herein, lasers with wavelengths within these two extremely strongly absorbed ranges will be referred to as water-absorbed lasers, and their wavelengths as water-absorbed wavelengths, as opposed to lasers with wavelengths in the visible and near-to mid-infrared range (e.g., 400 nm-2000 nm) that will be referred to as the deeper penetration lasers, with their wavelengths referred to as the deeper penetration wavelengths.

In water-absorbed laser procedures, it is the tissue's water content, not its pigment, that plays the role of an absorbing chromophore. The laser-induced temperature elevation is thus not limited to a particular pigment, such as melanin or hemoglobin, but to the superficially irradiated tissue layer with its thickness determined by the laser's extremely short optical penetration depth (about 1 µm).

Examples of water-absorbed laser systems are an Er:YAG laser (peak λ = 2.94 mm), Er:fiber lasers (peak λ = 2.8 - 2.94 µm), Er,Cr:YSGG (peak λ = 2.73 µm) and CO₂ lasers (peak λ = 9 to 11 µm). The corresponding optical absorption depths of these lasers in water are *d*ₒ ≈ 0.8 µm for Er:YAG, *dₒ ≈* 2.5 µm for Er,Cr:YSGG, *dₒ ≈* 0.8-2.5 µm for Er:fiber lasers and *dₒ ≈* 4-10 µm for CO₂ lasers.

Examples of deeper penetration lasers are the blue diode laser ( λ = 400-500 nm), green KTP laser (λ = 532 nm), green diode lasers (500-520 nm) and yellow diode and solid-state lasers (λ = 560-600 nm), near and mid-infrared diode and fiber lasers (700-2000 nm), ruby laser (λ = 690 nm), alexandrite laser (λ = 755 nm), Nd:YAG laser (λ = 1064 nm), Er:glass laser (1540 nm), Th:YAG laser (λ = 1940 nm) and Ho:YAG laser (λ = 2100 nm). It is to be noted that deeper penetration lasers include also wavelengths above 1000 nm which may also be absorbed predominantly by water, however to a smaller extent than the wavelength ranges from 2.5 µm to 3.5 µm, preferably from 2.7 µm to 3 µm, and from 8 µm to 11 µm, preferably from 9 µm to 11 µm.

During comprehensive multi-step treatments involving different laser wavelengths and pulse modalities, two steps may be commonly performed. One of the steps involves a light peel, i.e., a very superficial "cold" ablation of the skin requiring short (≤ 2 ms) laser pulses at above ablation threshold laser fluences. In facial and other dermal treatments, this step aims to correct epidermal pigmentation and dull skin. By laser energy absorption of water within cells, the tissue is heated up leading to vaporization or destruction of its surface layer(s). The expression "cold" refers to the fact that most of the laser energy which is delivered in addition to the energy required to reach ablation threshold, is used up for sustaining the ablation process, and not for further increasing the tissue temperature.

And the second step involves a repeated delivery of rapid sequences of sub-ablative laser pulses with typical sequence durations 0.1 s to 10 s. In dermal treatments, this "hotter" step results in deeper skin heating and aims at skin tightening and tissue regeneration.

Due to their strong absorption in human and animal tissues, water-absorbed lasers are extremely well suited for cutting, incision and excision of soft and hard tissues. This is a reason for the water absorbed lasers to be most suitable for the first, light peel step. On the other hand, the second deep heating, non-ablative step is most commonly performed with deeper penetration lasers due to their substantially higher ablation threshold fluence.

However, water-absorbed wavelengths can be also used for non-ablative skin and mucosa treatments. For example, during multi-step facial treatments both steps may be based on the same water-absorbed Er:YAG laser wavelength where each of the two steps require different Er:YAG laser pulses and pulse sequences.

Typically, the above light peel and deep heating steps are applied with a substantial time gap between each other since the laser device needs to be re-set and recalibrated in-between the two steps. This is due to the inability of standard PFMs to quickly change the pulse parameters from sub-ablative to ablative regime, which is the case even when the two steps are performed with the same water-absorbed laser. Another reason involves a concern that performing different treatment steps within a short time could lead to an over-treatment.

The active treatment time of multi-step procedures is therefore long since the total tissue surface needs to be treated twice, once for each step. Additionally, the laser output energy utilization is not optimal since the heat within the tissue that is generated during one of the steps gets substantially dissipated before the second step can benefit from it.

Therefore, there is still a need to improve methods and apparatus for treating tissue.

### 3. Summary of the Invention

This summary is provided to introduce a selection of concepts that are further described below in the detailed description. This summary is not intended to identify key features or essential features of the claimed subject matter.

According to a first aspect, the above need is at least in part met by an apparatus for repetitively emitting trains of laser pulses onto an organic tissue, each train of laser pulses including at least one laser pulse of a first type and at least one laser pulse of a second type. A time, that may be called d between a last one of the at least one laser pulse of the first type and a first one of the at least one laser pulse of the second type is no more than 500 ms, preferably shorter than 300 ms, more preferably shorter than 100 ms. The at least one laser pulse of the first type comprises a fluence below an ablation threshold of the organic tissue, preferably at least 10% below the ablation threshold. The at least one laser pulse of the second type comprises a fluence above the ablation threshold of the organic tissue, preferably at least 10% above the ablation threshold. Furthermore, the train of laser pulses starts with a pulse of the first type and/or ends with a pulse of the second type.

By ensuring that the delay between the last laser pulse of the first type and the first laser pulse of the second type is no more than 500 ms, physical and physiological effects produced by the first type of laser pulse can directly be exploited by the second type of laser pulses and vice versa. In one example, the pulses of the first type may be employed to smoothly heat up the tissue by raising the temperature within the tissue to an acceptable level. This level may be determined by a pain threshold of a human being. The pain threshold of humans is commonly found to be approximately 47°C ± 1,5°C without anaesthesia (and 51 ± 1.5 °C with topical anesthesia). See e.g., EP 4 046 585 A1 and US 12,226,152 B1 for details.

It has been determined by various experiments that the pain experienced by a patient depends predominantly on the final baseline temperature Tₛ after a pulse sequence has been delivered, but not on the (short-term) high temperature peaks Tₚ caused by individual pulses and their transients, providing that the temperature transients are short enough. In fact, the quickly decaying temperature transients with thermal exposure durations shorter than 100 msec may cause, on short time scales, tissue temperatures Tₚ above 70°C and for thermal exposures shorter than 1 msec even up to the tissue final "boiling" point of about 255°C without causing significant pain, providing that the long-term baseline tissue temperature Tₛ does not increase above a certain pain threshold.

By thus "setting the stage", the exposure to a subsequent high energy pulse may be reduced, e.g., less energy may be needed by the last pulse to ablate tissue. Thus, the risk of undesired damages to surrounding areas of the organic tissue can be mitigated.

Apart from that, the inventors found that, by using at least one ablative pulse after the pulses of the first type, the subjective pain threshold of a patient may be increased. In other words, the laser pulses of a first type may be applied to achieve a baseline temperature of the organic tissue up to a point where patient would normally feel pain, which is however not felt when applying the at least one ablative pulse after the pulses of the first type as outlined herein.

Thus, by combining the different pulse types into one "hybrid" pulse train, a substantial shortening of the treatment time can be achieved by the synergy between both pulses types without causing any additional pain to the patient. This allows for a more selective, pain-free and overall safer treatment.

The synergetic interaction between laser pulses of the first and the second type is expedited if the time d between a last laser pulse of the first type and a first laser pulse of the second type is below 500 ms, more preferably less than 300 ms, most preferably less than 100 ms, which are the typical times required for the effectiveness of the gate pain control mechanism in the spinal cord.

It is sufficient that a train of laser pulses comprises, e.g., only one non-ablative and one ablative pulse. However, the train of laser pulses may also comprise several non-ablative and/or several ablative pulses. However, it is preferred that each train of laser pulses starts with a pulse of the first type and/or ends with a pulse of the second type.

In general, no requirements are made to the shape of the pulses. However, in some cases the pulses may be of a triangular, rectangular, gaussian or freely oscillating shape (when looking at the pulse intensity over time).

The apparatus may be adapted to emit pulses of different fluences. In general, fluence can be regarded as a measure of delivered energy per area. The energy of a pulsed laser may be determined by several factors such as pulse power and pulse duration.

A fluence of the at least one laser pulse i of the first type may be understood to be a fluence of each pulse F₁₋ᵢ, as well as a cumulative fluence F₁ = Σ F₁₋ᵢ of the at least one laser pulse of the first type within a single train of laser pulses i. The fluences F₁₋ᵢ of pulses must be below the ablation threshold Fₜ such that no ablation occurs, taking into account also the increase of the baseline temperature T_{s,1} during a delivery of the pulse sequence of the first type, from which the temperature gets transiently increased to T_{p,1-i} by pulse fluences F₁₋ᵢ.

A fluence of the at least one laser pulse j of the second type may be understood to be a fluence of each pulse F₂₋ⱼ, whereas the fluence F₂₋ⱼ of each pulse must be above the ablation threshold fluence (F₂₋ⱼ > Fₜ₋ⱼ), with the cumulatively delivered fluence of pulses of the second type being equal to F₂ = Σ F_{2- j} . It is to be noted that the ablation fluence F_{t -j}, as required to result in ablation for each of the pulses j gets progressively reduced as the baseline temperature gets increased during the delivery of pulses of the first and second type. However, the influence of the baseline temperature increase on the reduction of the ablation threshold is relatively small since the permitted final baseline temperature increase is limited by the pain threshold temperature to below about 25 °C, which is substantially lower than the temperature increase of about 225 °C as required to reach the ablation temperature T_{abl}. Therefore, the ablation threshold fluence can in most cases be approximated by Fₜ₋ⱼ ≈ Fₜ where Fₜ is the single pulse ablation threshold in the absence of the baseline temperature increase.

The single pulse ablation threshold Fₜ may be approximated by hₐδ wherein δ is an optical penetration depth of the organic tissue at the wavelength of the pulse. hₐ is a specific ablation energy (J/mm³) of the organic tissue. For example, for soft tissue, hₐ may be within the range of 1*10⁴ J/cm³ to 2*10⁴ J/cm³, e.g., approximately 1.5 10⁴ J/cm³. For example, for an Er:YAG laser wavelength δ may be approximated by 1 µm. It should be noted that the ablation threshold may depend on the time interval during which the respective pulses and their fluences are delivered. For example, if energy is delivered with a time duration substantially shorter than the thermal relaxation time (TRT) of the organic tissue to prevent dissipation losses due to thermal diffusion, the ablation threshold is essentially an instantaneous ablation threshold for the fluence. The thermal relaxation time can be regarded as a characteristic time interval during which a thermal system returns to its equilibrium state. More precisely, in the case of a thermal elevation out of equilibrium, the TRT may be understood as the time during which the system cools down from 100% of the thermal elevation to 1/e ≈ 37% of the thermal elevation. In other words, TRT is the time after which the thermal system has lost its elevation by a factor of approximately 63%. If the energy is spread out in time over a longer duration, a higher energy may generally be needed to cause ablation, since a part of it dissipates into the surrounding tissue, e.g., due to thermal diffusion.

This time interval is referred to as energy delivery time that is understood to be the time during which a second half of energy is delivered (full width at half maximum for symmetric pulses).

Apart from the fluence, also various further pulse parameters may be used to distinguish the laser pulses of the first type and those of the second type. For example, pulse duration, pulse energy, (peak) power, repetition rate, wavelength, pulse shape and/or any other pulse (train) parameter. In some examples, however, they may only differ in terms of their fluence, e.g., by having a higher (peak) power but the same pulse duration and/or by having the same (peak) power but different pulse durations.

In some examples, the trains of laser pulses may be emitted by a (same) laser source of the apparatus. Specifically, in one example, the apparatus according to the first aspect may comprise a single laser source which emits the trains of laser pulses. Specifically, the laser pulses of the first and second type may comprise a same wavelength, but may differ in peak power, pulse duration and/or optionally pulse shape. The laser source may be an Er:YAG or CO₂ laser source or any other water-absorbed laser source emitting light with a wavelength close to a water absorption maximum.

In general, however, the laser pulses of the first and of the second type need not have the same wavelength. For example, an Nd:YAG source (or any other source with a deep penetration wavelength) emitting non-ablative pulses of the first type may be used, e.g., to pre-heat the organic tissue including deeper layers. The number of pulses of the first type in a single pulse train may be varied. An Er:YAG or CO₂ laser source may be used to emit the at least one laser pulse of the second type having an ablative effect on the skin surface.

In some examples, the apparatus may comprise a mode which can be selected (e.g., out of a plurality of individual modes), wherein selecting the mode causes the apparatus to automatically emit the train of laser pulses.

The apparatus may be configured such that a time between a last one of the at least one laser pulse of the second type of a train and a first one of the at least one laser pulse of the first type of a subsequent train is no more than 500 ms, preferably shorter than 300 ms, more preferably shorter than 100 ms. Thus, the time between subsequent pulse trains may be tailored as needed. In some examples, a pulse train may end with a pulse of the second type and begin with a pulse of the first type. The time between the last pulse of the second type of a pulse train and the first pulse of the first type of the subsequent pulse train may also be referred to as pulse train separation time. The pulse train separation time may in some examples be as long as the time d between the last one of the at least one laser pulse of the first type and the first one of the at least one laser pulse of the second type within a pulse train. In other examples, the pulse train separation time may be longer, e.g., at least twice, at least five times, or at least ten times as long as the time d.

In another example, the apparatus may be configured so that the at least one laser pulse of the first type forms a first group of subsequent laser pulses (i =1, 2, ... N₁), and/or wherein the at least one laser pulse of the second type forms a second group of subsequent laser pulses (j = 1, 2, ..., N₂). Thus, a train composed of a sequence of pulses of the first type which may be emitted before any pulse of the second type is emitted. A number of 1, 2, ..., N₁ pulses of the first type may precede a number of 1, 2, ..., N₂ of the second type. In this example, the train of pulses may comprise two distinct sub-trains, each comprising pulses of a single type. In other examples, also more than two distinct sub-trains or an alternating sequence of two distinct sub-trains may be comprised by a train of laser pulses.

The train of laser pulses may comprise an alternating sequence of these first and second groups, for example. The term "alternating sequence" (or "alternating sub-trains") may be understood to comprise or be a sequence A-B-A, wherein A comprises one or more laser pulses of the first type and B one or more laser pulses of the second type. Another sequence could be A-B-A-B. Likewise, B-A-B or B-A-B-A would also qualify as such alternating sequences. It is important to note that the time delay between the last laser pulse of any sequence A and the first laser pulse of any sequence B may in some examples not exceed 500 ms. Vice versa, the time delay between the last laser pulse of a sequence B and the first laser pulse of any sequence A may not exceed 500 ms. In a longer sequence A-B-A-B (...) this condition may be satisfied between every single sequence A and B and between any sequence B and A.

In a further example, the apparatus may be configured such that the at least one laser pulse of the first type comprises more than 2 and less than 150 laser pulses of the first type, preferably with a repetition rate of 10 to 100 Hz. Additionally or alternatively, the at least one laser pulse of the second type may be a single laser pulse of the second type.

In this configuration, particularly advantageous outcomes may be achieved. For example, the organic tissue may be heated by the first group of subsequent laser pulses to a temperature T_{s,1}, such as the human pain threshold of approximately 47°C (or slightly above). During this time, no ablative effect is provided. A subsequent high energy pulse may be emitted to the pre-heated skin. As outlined, this additional pulse may be particularly helpful to reduce pain as felt by the patient and thus make the treatment less-painful or allow higher treatment effect/temperature without any increased pain. In other words, the laser pulses of the first type may be applied to achieve a baseline temperature of the organic tissue up to a point where patient would normally feel pain, which is however not felt when applying the at least one ablative pulse after the pulses of the first type as outlined herein. This effect is currently explained in view of a potential blocking effect of the ablative pulse on the skin surface, blocking nerve stimulations transmitting the sensation of pain to the brain. A blocking nerve stimulation effect may occur when the first step's thermal and the second step's ablatively caused tactile stimulations are generated in quick succession. Another benefit of delivering non-ablative and ablative steps in quick succession is that in this manner the practitioner can visually track from clearly visible ablated traces on the tissue whereas otherwise difficult-to-observe non-ablative steps had been delivered.

In some examples, generally a plurality of pulses of a first type may be combined with a single laser pulse of the second type.

The laser pulses of the first type may, in some examples, all be equal in peak power, energy, and pulse duration. Additionally or alternatively, in some examples, they may have a fixed repetition rate (i.e., within a train of pulses, the laser pulses of the first type may be repeated according to a constant time period).

Preferably, especially when the same laser source (i.e., wavelength) is used to generate pulses of the first and second type, the second type of laser pulse is adapted to have a (significantly) higher peak power level than any laser pulse of the first type, for example, at least 10%, at least 25% or at least 50% higher. The second laser pulse type may additionally or alternatively also be shorter than the first laser pulse type. This may be preferable if both types use the same laser wavelength.

In another example, a plurality of laser pulses of the first type may be combined with two subsequent laser pulses of the second type. As for the case of a single laser pulse of the second type, these laser pulses of the second type may be adapted to have a (significantly) higher peak power level than any laser pulse of the first type, for example, at least 10%, at least 25% or at least 50% higher. The two laser pulses of the second type may additionally or alternatively be shorter than the first laser pulse type.

In some examples, each of the laser pulses of the second type may comprise a fluence such that ablation is achieved, e.g., with a fluence above the corresponding ablation threshold preferably at least 10% above.

In some examples, the at least one laser pulse of the first type comprises a plurality of laser pulses of the first type, and the time d corresponds to an inverse of a (mean) repetition rate of the plurality of laser pulses of the first type. This configuration may make it particularly easy to implement the pulse train, as the source may be used at the same repetition rate for first and second pulse types, such that re-setting can be avoided in a simple manner. Also, this criterion may ensure that synergetic effects between pulses of the first type and pulses of the second type can be achieved. For example, the inverse of the repetition rate of the plurality of laser pulses of the first type may be less than 500 ms, less than 100 ms, less than 50 ms or less than 10 ms. In some examples it may be at least 1 ms or at least 10 ms.

The time between subsequent pulse trains may in some examples correspond to one-time, two times, three times, four times, or any other integer multiple of the inverse of a repetition rate of the plurality of laser pulses of the first type.

In some examples, the apparatus may be adapted to deliver at least one train of the laser pulses onto a same area of the organic tissue. This may also be referred to as "stamping" or "stacking" herein. The apparatus may for example comprise a scanner or a robot which automatically adapts the apparatus such that the corresponding laser pulses are delivered to the desired location of the organic tissue. For example, a pulse map may be stored by the apparatus, and the apparatus automatically moves the position of the laser pulses such that the pulses are applied to the organic tissue according to the pulse map. The area of the organic tissue to which pulses are to be applied may be decomposed into a plurality of positions, at each of which one or more laser pulse trains are applied. The pulse trains to a specific region may be applied right after each other. However, in some examples, a larger region, e.g., the entire region to be treated, is first treated with one pulse train, and then the sequence is repeated.

In other examples, the apparatus may be adapted to deliver pulses of a train of the laser pulses onto at least partially different areas of the organic tissue. This may be referred to as a "brushing" mode herein. As explained with reference to the "stamping" mode, the apparatus may comprise a scanner or a robot which automatically adapts the apparatus such that the corresponding laser pulses are delivered to the desired location of the organic tissue. The same aspects may apply to the "brushing" mode.

For example, in the "brushing" mode, the position of the tissue onto which the laser pulses are applied undergo a constant uniform motion. In this case, for example, a number N₁ (e.g. = 6) of pulses of the first type is followed by a single pulse of the second type, wherein the pulses follow each other with a fixed repetition time (e.g. tₛₑᵣ = 50 ms), resulting in a continuous sequence of successive trains of laser pulses, i.e., one pulse every 50 ms.

In some examples, the apparatus may be adapted for skin tightening, skin rejuvenation, removal of wrinkles of skin, or similar aesthetic applications.

In one configuration, an apparatus may be adapted to deliver pulses such that a cumulative fluence of the at least one laser pulse of the first type (of a pulse train) is less than 2.0 J/cm², e.g. for Er:YAG lasers. In other examples, a cumulative fluence of the at least one laser pulse of the first type (of a pulse train) may be provided by the apparatus such that the organic tissue is heated to a target temperature below a pain threshold temperature. In some examples, the cumulative fluence may be adapted such that the pain threshold temperature is met, e.g., by ±5°. In some examples, the target temperature is defined as that (peak) baseline temperature T_{s,1} of the organic tissue after the last pulse of the first type (of a pulse train) has been applied, e.g., after a mean repetition time of the pulses of the first type. For example, if the pulses are applied at a certain (constant) repetition time, the target temperature may be understood as the (peak) baseline temperature after application of the last pulse of the first type, namely one repetition time after application of that pulse. If varying repetition times are used for the pulses of the first type, it may be at a mean repetition time after application of the last pulse of the first type.

In another aspect of the invention, the at least one laser pulse of the second type is adapted such that an ablation depth of the at least one laser pulse in the organic tissue is greater than a diffusion depth of the heat provided by the at least one laser pulse in the organic tissue.

In the context of this invention, the ablation depth is understood to be the depth of material removed per laser pulse. In many cases, the material may be (a) skin layer(s), most often the stratum corneum as uppermost layer. In some examples, other parts of a human or animal body may be targeted, such as mucosa. In general, the ablation depth may be affected by the wavelength, pulse power, pulse duration, repetition rate and consequently, by the fluence.

The diffusion depth is to be understood as a characteristic depth at which heat penetrates into a material. In general, Fourier's Heat Equation governs heat diffusion ∂*T*/∂*t = DΔT,* Δ denoting the Laplace operator and D the heat diffusivity (approximately 0.1 mm² s⁻¹ for organic soft tissue). The diffusion depth may then be approximated by ${d}_{diff} ∼ \sqrt{{Dt}_{p}}$*,* where *tₚ* is the pulse duration.

A cooling process within organic tissue may in some examples be predominantly caused by a relatively fast diffusion in the longitudinal, i.e., vertical direction of the heat from the thin superficially irradiated tissue layer into the underlying deeper tissue layers, and to a smaller extent also by a convection into the surrounding air or other surrounding media.

For a planar geometry, the depth of longitudinal thermal diffusion during a laser irradiation of duration tₚ can be approximated by ${d}_{diff , long} \approx 2 \sqrt{{Dt}_{p}}$,

It may be noted that the thermal diffusion in the transverse direction, i.e., radially away from the laser-irradiated spot, is also present. The transverse heat diffusion length can generally be approximated based on laser irradiation time tₚ according to ${d}_{diff , trans} \approx 4 \sqrt{{Dt}_{p}}$*.* For tp =1 ms, the longitudinal and transverse diffusions lengths are equal to 21 µm, and 42 µm, respectively. The longitudinal diffusion length is thus longer than, for example, the optical absorption depth of Er:YAG, resulting in a substantial diffusion cooling already during a single laser pulse. On the other hand, the transverse diffusion length is substantially smaller than a typical laser spot diameter of 0.25-30 mm, resulting in a negligible effect of transverse diffusion for single superficially ablative laser pulses.

For typical pulse trains, pulse train durations of the first type of pulses (t₁) may be from 0.1 s to 5 s. The corresponding longitudinal and transverse diffusion lengths for t₁ = 0.3 s are equal to 0.36 mm and 0.72 mm, respectively. The longitudinal diffusion length thus has an even more pronounced effect on the heating dynamics than in the single pulse case, while the transverse diffusion has a significant influence on the heating dynamics only for laser diameters (d') smaller than about 1.5-2 mm. It should be appreciated that for laser spot dimensions d' ≤ 1.5 mm, the thermal diffusion in the lateral (i.e., transverse) direction(s) during the time span t_{sep,1}, where t_{sep,1} represents the time in-between individual pulses in a sequence, can be appreciable, which contributes to the rate of temperature decay during the time span between two laser pulses. Therefore, for d' ≤ 1.5 mm, cooling times will be shorter due to lateral diffusion. Thus, using a small spot size allows the delivery of higher fluences at shorter serial times t₁. For this reason, according to one of the preferred embodiments of present invention, the energy is delivered to the tissue in a patterned shape. This patterned illumination may generally comprise a polka-dot lattice in particular, the pattern properties as described in EP 3 569 287 A1 which is incorporated by reference in full. In other embodiments, the illumination may comprise other illumination patterns, for example a ring or a honeycomb lattice pattern, as described in EP 23174965.6 which is incorporated by reference in full.

Beam spot sizes with d' ≥ 3 mm are in turn characterized by their heating dynamics not being substantially affected by the transverse heat diffusion.

The configuration of the ablation depth being greater than the diffusion depth may ensure that the ablation remains essentially "cold". Under these circumstances, the delivered energy is predominantly used for the ablation of the pre-heated tissue, with only a very thin tissue layer within the optical absorption depth being heated up to the ablation temperature. If the ablation depth were smaller than the diffusion depth, deeper layers of the organic tissue would continue to be heated by the effects resulting from thermal diffusion, possibly leading to pain and/or undesired damages of these layers.

In some examples, the apparatus may be adapted such that the at least one laser pulse of the second type comprises an ablation depth in the organic tissue below 100 µm, preferably below 50 µm, more preferably below 30 µm, most preferably below 10 µm. This may ensure that only superficial layers are removed. It has turned out that such relatively shallow ablation is sufficient for achieving the pain reduction effect as described herein. Specifically, for numerous medical and/or purely aesthetic/cosmetic treatments targeting skin, it may be sufficient to ablate only the stratum corneum layer which mainly consists of dead flattened skin cells. By restraining the treatment to this area, also pain and undesired damages to lower skin layers may be prevented.

In one example, the apparatus may be configured such that the at least one laser pulse of the second type comprises a fluence between 2.0 and 10 J/cm². Preferably, to this end, pulse durations for pulses of the second type may be in the range of 1 µs to 1 ms, preferably 10 µs to 500 µs, most preferably 150 µs to 300 µs.

In some exemplary configurations of the apparatus, a pulse length of the at least one pulse of the second type may be shorter than a pulse length of the at least one laser pulse of the first type, preferably shorter than 600 µs, more preferably shorter than 400 µs, most preferably shorter than 330 µs, or even 250 µs. This may limit the diffusion depth of the pulses of the second type, and thus, inter alia, facilitate "cold" ablation.

In some examples, the at least one laser pulse of the first type and/or the at least one laser pulse of the second type may be adapted such as to illuminate the area of the organic tissue with an illumination pattern having an effective surface coverage (C) of more than 10% and less than 80 % of the area of the organic tissue. In some examples, the effective surface coverage may be up to 50%, 40 %, 30%, or up to 20%. Limiting the surface coverage increases transversal diffusion processes (parallel to the surface of the organic tissue). This may be advantageous, as it may limit the vertical diffusion depth described herein.

A pattern can comprise a plurality of laser spots arranged with gaps between one another. A pattern may comprise a lattice of spots. In some examples, rings and/or other line structures may be provided, e.g., hexagons.

In each case, an outmost (and shortest) laser illumination contour may be defined by a pattern, and the tissue surface coverage C may be defined as the ratio of illuminated area and total area encircled by the outmost laser illumination contour.

At 100% surface coverage, heat dissipation would predominantly only take place towards deeper tissue and only around the contour of the covered surface in lateral directions (dissipation to air is usually minor compared to the internal diffusion). Hence, by using surface coverage in the mentioned ranges, additional heat gradients in lateral direction between the illuminated "spots" or "lines" are produced. This may expedite the cooling process, enabling higher peak temperatures on the illuminated spots which decay relatively quickly. At the same time, a relatively large surface coverage ratio is still ensured allowing for short treatment times.

In some examples, the illumination pattern may comprise a honeycomb lattice (comprising a plurality of hexagonal contours) and/or a ring lattice (comprising a plurality of rings) and/or a polka dot lattice (comprising a plurality of dots).

For example, the ring lattice may comprise rings with a radius in the range of 0.01 to 1 mm and/or a line width in the range of 0.05 to 0.3 mm. For example, the honeycomb lattice may comprise hexagons with edge lengths in the range from 0.1 to 3 mm, preferably from 0.75 mm to 1.25 mm or from 0.05 to 2 mm and/or line widths in the range from 0.065 mm to 0.3 mm, preferably from 0.1 mm to 0.2 mm, yielding surface coverages of 10% to 52% and 16% to 33%, respectively. Similarly spot patterns may be used with spot diameters in the range from 0.01 to 2 mm, preferably from 0.5 to 1.5 mm, or from 0.01 to 0.3, or from 0.1 to 0.2.

In some examples, the apparatus may be adapted to selectively illuminate the area of the organic tissue with two or more illumination patterns. For example, the apparatus may be adapted to work with two different handpieces that may be used manually and/or attached to a scanner of the apparatus. The apparatus may be adapted to configure the time (d) between the last laser pulse of the at least one laser pulse of the first type and the first laser pulse of the at least one laser pulse of the second type according to the illumination pattern, e.g., selected for the first type of laser pulses. This may allow to achieve a desired thermal interaction between first and second pulse types. Since the type of illumination pattern may determine how quickly the illuminated areas cool down, adjusting the time between first and second pulse types may allow to realize a certain desired thermal effect across various illumination patterns.

In some examples, the apparatus may comprise at least two predetermined modes selectable by the user, e.g., via a user interface that may comprise a touch screen or similar input device(s). At least one mode may comprise emitting a train of laser pulses as described herein. In some examples, at least one mode may comprise emitting a train of laser pulses in which all pulses are identical. In some examples, the user interface may, optionally, allow the user to predetermine properties of the first type of pulses, properties of the second type of pulses, and/or a time (d) between the last pulse of the first type and the first pulse of the second type of the at least one mode.

In some examples, the apparatus may comprise at least two predetermined modes with a sequence of laser pulses of a first type being patterned. In the first mode, the pulses of the first type may comprise a first pattern, and the time between the last laser pulse of the sequence of laser pulses of the first type and the first laser pulse of the at least one laser pulse of the second type may comprise a first value. In the second mode, the pulses of the first type may comprise a second pattern, and the time between the last laser pulse of the sequence of laser pulses of the first type and the first laser pulse of the at least one laser pulse of the second type may comprise a second value. Thus, the apparatus may automatically take the geometry of the pattern and its surface coverage into account to configure the time between two different laser pulse types.

In some examples, the apparatus may be adapted to calculate and/or display a final (target) temperature based on at least one selected parameter of the train on laser pulses and/or an illumination pattern for pulses of the first and/or second type. The target temperature may comprise a target temperature after application of the laser pulses of the first type, and/or a target temperature after application of the at least one laser pulse of the second type. To this end, the apparatus may comprise a corresponding user interface that may comprise a touch screen or similar input/output device(s). In some examples, instead of displaying and/or calculating a final (target) temperature, the apparatus may calculate and/or display a corresponding parameter relating to the final (target) temperature, e.g., depending on the desired treatment effect.

The target temperature after application of the at least one laser pulse of the second type may be understood as a temperature after application of the last one of the laser pulses of the second type, specifically after a (mean) repetition time of all preceding pulses of the respective pulse train.

In some examples, the apparatus may be adapted such that the user can select a parameter relating to the target temperature, e.g., depending on the desired treatment effect. For example, a relatively low intensity, medium intensity, or high intensity treatment may be desired. The apparatus may then determine the parameters of the first type of laser pulses accordingly, e.g., such that a target temperature after application the pulses of the first type is below a pain threshold temperature (e.g. in the range of 40° to 45°), around the pain threshold temperature (e.g. in the range of 45° to 50°), or above the pain threshold temperature (e.g., in the range of 50° to 55°). The apparatus may further determine the time delay d and the parameters of the second type of pulse(s) accordingly. For example, if the target temperature after application of the first type of pulses is higher, the fluence of the second type of pulses may be lowered.

In some examples, the apparatus may comprise means for actively cooling the surface of the tissue, e.g., a spray cooling that directs a mixture of liquid and gas onto the surface. In some examples, the apparatus may be configured such that the spray cooling is automatically scanned together with the pulses such that the areas onto which the pulses are directed are selectively cooled.

In other examples, a wavelength of the at least one laser pulse of the second type and/or a wavelength of the at least one laser pulse of the first type comprises a water-absorbed wavelength, preferably an Er:YAG laser wavelength. This may be beneficial for reaching a superficial heating and/or ablation effect on the organic tissue. Er:YAG lasers may generally provide wavelengths in the range of about 2.6 µm to 3 µm, respectively.

In general, also various other laser sources may be used to supply the pulses as described herein, e.g. with a wavelength in the infrared, e.g. mid-infrared spectrum. For example, CO₂ lasers may be used that may provide a wavelength within the range of 9.3 to 10.6 µm. Among mid-infrared lasers, the laser wavelengths most suitable for water-mediated treatments are located at the two highest water absorption peaks, corresponding to a first range from 2.5 µm to 3.5 µm, preferably from 2.7 µm to 3 µm, and to a second range from 8 µm to 11 µm, preferably from 9 µm to 11 µm.

Other conceivable laser sources may comprise quantum cascade lasers to produce laser light in the mid-infrared region of the spectrum, typically between 4 µm and 12 µm. Semiconductor lasers, such as based on gallium arsenide or indium phosphide, may be used to produce laser light with a wide range of wavelengths, including the infrared region.

In some examples, also Nd:YAG lasers with a wavelength of around 1000 nm to 1150 nm may be used, particularly for the first type of pulses. Another type of laser that may be used is a blue diode laser ( λ = 400-500 nm), green KTP laser (λ = 532 nm), green diode laser (500-520 nm) and yellow diode and solid-state laser (λ = 560-600 nm), near and mid-infrared diode and fiber lasers (700-2000 nm), ruby laser (λ = 690 nm), alexandrite laser (λ = 755 nm, Er:glass laser (1540 nm), Th:YAG laser (λ = 1940 nm) and Ho:YAG laser (λ = 2100 nm), for examples.

According to another aspect, the above need may also, at least in part, be met by a method for treatment of an area of an organic tissue. The method may comprise: emitting trains of laser pulses onto an organic tissue, each train of laser pulses including at least one laser pulse of a first type and at least one laser pulse of a second type. A time d between a last one of the at least one laser pulse of the first type and a first one of the at least one laser pulse of the second type is no more than 500 ms, preferably shorter than 300 ms, more preferably shorter than 100 ms. At least one laser pulse of the first type comprises a fluence below an ablation threshold of the organic tissue, preferably at least 10% below the ablation threshold. At least one laser pulse of the second type comprises a fluence above the ablation threshold of the organic tissue, preferably at least 10% above the ablation threshold. The train of laser pulses starts with a pulse of the first type and/or ends with a pulse of the second type.

In some examples, it is also possible that one or more laser pulses of a third, fourth, ..., etc., type are emitted in between the pulses of the first type and the second type. For example, the last laser pulse of the sequence of pulses of the first type may be followed by one or more pulses of a third type, that has an increased peak power level compared to the first type, but a lower peak power compared to the second type. For example, the pulses of the first and third type may be separated also by a time below 500 ms. In some examples, it may also be possible that there is no additional temporal separation, but the first and second (and possibly third) type pulses are delivered consecutively, e.g., with a constant pulse period.

In another aspect, the apparatus as described before may be adapted to obtain a cumulative fluence of the first group of subsequent laser pulses (1, 2, ..., N₁), and to automatically determine a duration of the first group of subsequent laser pulses (1, 2, ... N₁) such that the organic tissue is heated to a predetermined target temperature by the cumulative fluence F₁ of the first group of subsequent laser pulses.

This may be done by employing means for determining (e.g., a control unit) adapted to determine the duration t₁ and/or the cumulative fluence F₁ of the laser pulses of the first type such that $\left(1-{x}_{1}\right) {ΔT}_{p} / \left({A}_{s} x {{t}_{1}}^{- Ks}\right) < {F}_{1} < \left(1+{x}_{2}\right) * {ΔT}_{p} / \left({A}_{s} x {{t}_{1}}^{- Ks}\right) ,$ wherein ΔTₚ is a pain threshold temperature increase (e.g., 22°C), x₁ and x₂ (are margin values; e.g., x₁ = 0.5, x₂= 0.5, and/or x₁ = 0.2, x₂=0.2), and wherein Kₛ and Aₛ are predetermined parameters, depending on the tissue type and/or the laser source. The above range for F₁ is based on the insight, that the (target) temperature increase created on target tissue by application of a laser pulse sequence is approximately proportional to the cumulative fluence F₁, and can, more specifically be described by ΔTₛ = Aₛ x Fₛ x t₁ ^{-Ks}. This temperature increase is to be below, around, or above a pain threshold temperature increase ΔTₚ.

The right inequality (F₁ < (1+x₂) * ΔTₚ /(Aₛ x t₁^{-Ks})) takes into account a margin x₂. In some examples, ΔTₚ may be used as indicated above for specific tissue types (e.g., 12.7 °C for skin (no anaesthesia), etc.). x₂ may also be selected to be zero in the above formulas, or even to be negative to allow for a safety margin, e.g. -0.05, -0.1, or -0.15, or -0.2 (which corresponds to a limit of 80% or 85% or 90% or 95% of the respective specific threshold temperature increase).

The left inequality ((1-x₁) ΔTₚ /(Aₛ x t₁ ^{-Ks}) < F₁) is to ensure that the effect of the laser pulse sequence has a certain minimum effect. The final temperature increase shall be at least (1-x₁) ΔTₚ. Also here, a uniform threshold may be used regardless of the tissue type using a margin value x₁. However, also a value specific for the tissue type (e.g. as outlined above) may be used. In some examples, x₁ may be 0.5, 0.4, 0.3, 0.2, or 0.1, 0.05, or even 0 (to provide at least 50%, 60%, 70%, 80%, 90%, 95% or 100% of the threshold temperature increase).

For example, an apparatus may comprise an Er:YAG laser source for generating the laser pulse sequence. The means for determining (e.g. control unit) may then be adapted as outlined above wherein Kₛ is within the range 0.3 to 0.5, 0.35 to 0.45, or approximately 0.4. As may then be a predetermined parameter, wherein the means for determining may use and/or store (in a permanent memory) a value for this parameter depending on the tissue type and/or depending on whether the beam of the laser pulse sequence is to be pixelated.

For example, for an Er:YAG laser source, the means for determining may be adapted to determine the duration t₁ (in units of ms) and/or the cumulative fluence F₁ (in J/cm²) of the pulse sequence such that $\left(1-{x}_{1}\right) {ΔT}_{p} / \left({A}_{s} x {{t}_{1}}^{- Ks}\right) < {F}_{1} < \left(1+{x}_{2}\right) * {ΔT}_{p} / \left({A}_{s} x {{t}_{1}}^{- Ks}\right) ,$ wherein ΔTₚ is 22°C; and x₁= 0.5, x₂=0.5 or any other values described herein. Kₛ may be equal to 0.43.

Aₛ = 84 (°C cm²)/J, if human skin is the tissue type and no pixelization of the laser pulse sequence is to be used (e.g. as indicated by corresponding selection input); Aₛ = 69 (°C cm²)/J, if human mucosa is the tissue type and no pixelization 25 of the laser pulse sequence is to be used (e.g. as indicated by corresponding selection input); Aₛ = 28 (°C cm²)/J, if human skin is the tissue type and pixelization of the laser pulse sequence is to be used (e.g. as indicated by corresponding selection input ); Aₛ = 18 (°C cm²)/J, if human mucosa is the tissue type and pixelization of the laser pulse sequence is to be used (e.g. as indicated by corresponding selection input).

The above values for Aₛ are exemplary only, and in other examples, different values may be used, e.g. with Aₛ selected from the range 80 to 90 or 82 to 86, instead of 84. Also, Aₛ may be selected from the range 50 to 60 or 53 to 57, instead of 55. Also, Aₛ may be selected from the range 24 to 34, or 26 to 30, instead of 28. Finally, Aₛ may also be selected from the range 14 to 24, or 16 to 20, instead of 18.

In some examples, the means for determining may be adapted to determine the duration t₁ and/or the cumulative fluence F₁ of the first group of subsequent laser pulses such that (1-x₁) ΔTₚ /(Aₛ x t₁ ^{- Ks}) < F₁ < (1+x₂) * ΔTₚ /(Aₛ x t₁ ^{-Ks}), wherein x₁ = 0.4, 0.3, 0.2, 0.1. or 0.05.

In some examples, the means for determining may be adapted to determine the duration t₁ and/or the cumulative fluence F₁ of the first group of subsequent laser pulses such that (1-x₁) ΔTₚ /(Aₛ x t₁ ^{- Ks}) < F₁ < (1+x₂) * ΔTₚ /(Aₛ x t₁ ^{- Ks}), wherein x₂ = 0.4, 0.3, 0.2, 0.1, 0.05, 0, -0.05, or -0.1. In other examples, x₁ and/or x₂ may be selected from the range 0 to 0.5, 0 to 0.3 and/or 0.1 to 0.3.

Additionally or alternatively, for the right inequality (F₁ < (1+x₂) * ΔTₚ /(Aₛ x t₁ ^{-Ks})), ΔTₚ may be used as indicated above for specific tissue types (e.g. 12.7 °C for skin (no anaesthesia), etc.). x₂ may then be selected to be zero, or even to be negative to allow for a safety margin, e.g. -0.05, -0.1, or -0.15, or -0.2. Also in the left inequality, ΔTₚ may be used as indicated above for specific tissue types, wherein x₁ may be 0.5, 0.4, 0.3, 0.2, or 0.1, 0.05, or even 0.

Further the apparatus as described before may be adapted to obtain a duration of the first group of subsequent laser pulses (1, 2, ... N₁) and to automatically determine a cumulative fluence of the first group of subsequent laser pulses (1, 2, ..., N₁) such that the organic tissue is heated to a predetermined target temperature by the cumulative fluence Fₛ of the first group of subsequent laser pulses. The relations between duration and cumulative fluence as outlined herein may be used as well.

Further aspects relating to the automatic determination of a fluence and/or duration of the first group of subsequent laser pulses may be implemented as described in European patent application EP 4 046 585 A1 which is incorporated herein by reference.

Generally, functionalities described herein may be provided in hard- and/or software.

For example, a computer program may be provided that comprises executable instructions to cause an apparatus, for example, as described herein, to automatically implement the method steps and/or the various further functions described herein.

Whether aspects or functions of the device are implemented as hardware or software depends upon the particular application and design constraints imposed on the overall system. By way of example, an element, or any portion of an element, or any combination of elements may be implemented as a processing system that may include one or more processors. Examples of processors include microprocessors, microcontrollers, graphics processing units (GPUs), central processing units (CPUs), application processors, digital signal processors (DSPs), reduced instruction set computing (RISC) processors, systems on a chip (SoC), baseband processors, field programmable gate arrays (FPGAs), programmable logic devices (PLDs), state machines, gated logic, discrete hardware circuits, and other suitable hardware configured to perform the various functionality described throughout this disclosure.

One or more processors in the processing system may execute software. Software shall be construed broadly to mean instructions, instruction sets, code, code segments, program code, programs, subprograms, software components, applications, software applications, software packages, routines, subroutines, objects, executables, threads of execution, procedures, functions, etc., whether referred to as software, firmware, middleware, microcode, hardware description language, or otherwise.

Accordingly, in one or more exemplary embodiments, the functions described may be implemented in hardware, software, or any combination thereof. If implemented in software, the functions may be stored on or encoded as one or more instructions or code on a computer-readable medium. Computer-readable media includes computer storage media. Storage media may be any available media that can be accessed by a computer. By way of example, and not limitation, such computer-readable media can comprise a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that can be used to store computer executable code in the form of instructions or data structures that can be accessed by a computer.

It is noted that any combination of features that have been described above as belonging to certain embodiments/aspects of the present invention may also form an embodiment of the present invention.

### 4. Short description of the figures

Fig. 1 shows an exemplary train of laser pulses comprising different types of laser pulses that differ in pulse duration, repetition rate and peak power level;
Fig. 2 shows a further exemplary pulse train. The first part comprises a sequence of six non-ablative laser pulses. The second part comprises only one single ablative laser pulse of a shorter pulse duration and a higher peak power level;
Fig. 3 shows an exemplary pulse train and its effect on the tissue temperature. The first part comprises a sequence of seven non-ablative pulses, and the second part comprises two ablative laser pulses of a shorter pulse duration and a higher peak power level;
Fig. 4 shows the effect on the tissue temperature of a further exemplary pulse train for equally distant laser pulses; The first part comprises a sequence of six non-ablative pulses, and the second part comprises one ablative laser pulse of a shorter pulse duration and a higher peak power level;
Fig. 5 shows a diagram that exemplifies the relation between temperature increase due to an ablative pulse and fluence of an ablative pulse;
Fig. 6 shows an exemplary heat distribution in an organic tissue after illumination with a laser pulse;
Fig. 7 shows different illumination patterns;
Fig. 8 shows a diagram that exemplifies the relation between temperature increase due to an ablative pulse and fluence of an ablative pulse for the case of a full beam laser irradiation and a polka dot laser irradiation;
Fig. 9 shows pain measurements according to the VAS method in dependence of the fluence of a laser pulse of the second type.

### 5. Detailed description

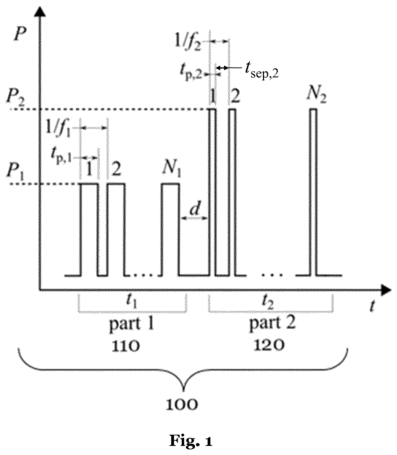

Fig. 1 shows a first example for a train of laser pulses 100 including at least one laser pulse 110 of a first type and at least one laser pulse 120 of a second type. The pulses are shown as comprising a rectangular temporal pulse shape (flat top) in terms of power P as a function of time t. However, this is just exemplary, and the pulses may generally comprise a Gaussian distributed shape, a left-shifted shape, a right-shifted shape, a free-laser-generation shape, etc., wherein the temporal pulse shape of the first and second types do not necessarily have to coincide.

The number of laser pulses 110 of the first type may be 1, 2, ... or generally any number N₁. The laser pulses i = 1,...N₁ of the first type may comprise a first pulse duration t_{p,1}, and a first peak power P₁, and they may be separated by a first pulse period 1/f₁, wherein f₁ may be the repetition rate of the pulses of the first type. The separation time, i.e. the time between the end of a pulse of a first type and the beginning of a subsequent pulse of a first type may then be equal to t_{sep,1} = 1/f₁ - t_{p,1}. The total duration of the partial pulse train 110 including the pulses of the first type may be t₁ (given by t₁ = N₁/*f*₁.)^{.}

After a time d between the end of the last pulse i = N₁ of the first type 110, the first one (j = 1) of the second type of pulses 120 may start. The time d may be adapted as described herein, in particular, it may be no more than 500 ms. In some examples, it may be less than 300 ms or less than 100 ms, for example.

The number of laser pulses 120 of the second type may be 1, 2, ... or generally any number N₂ which may or may not differ from N₁. The laser pulses j = 1,...,N₂ of the second type may comprise a second pulse duration t_{p,2}, and a second peak power P₂, and they may be separated by a second pulse period 1/f₂, wherein f₂ may be the repetition rate of the pulses of the second type. The separation time, i.e. the time between the end of a pulse of a second type and the beginning of a subsequent pulse of a second type may then be equal to t_{sep,2} = 1/f₂ - t_{p,2}. The total duration of the partial pulse train 120 including the pulses of the second type may be t₂ (t₂ = N₂/f₂). At least one parameter of the laser pulses 120 of the second type differs from a corresponding parameter of the laser pulses 110 of the first type.

For example, and as shown in Fig. 1, the second pulse duration t_{p,2} may be shorter than the first pulse duration t_{p,1}, the second peak power P₂ may be higher than the first peak power P₁, and the second pulse period 1/f₂ may be shorter than the first pulse period 1/f₁. In other examples, the first and second type of pulses may additionally or alternatively differ in other respects, such as temporal pulse shape. In further examples, the first and second type of pulses may differ only in one or two respects.

An exemplary laser in the apparatus may emit Er:YAG laser pulses that are non-ablative, and ablative Er:YAG laser pulses to induce a light peel of the skin with single pulse parameters chosen (slightly) above the ablation threshold.

In general, the invention allows for various settings of the parameters of the respective laser pulses. However, the fluences F₁₋ᵢ of the pulses i of the first type do not exceed the ablation threshold, whereas the fluences F₂₋ⱼ of the pulses of the second type exceeds this threshold. In the example of Fig. 1, this amounts to the number N₁ of laser pulses delivered during t₁ not exceeding this fluence threshold. The pulses of the second type have fluences above the ablation threshold. Preferably, t_{p,2} is shorter than the TRT of the organic tissue (for human skin irradiated by an Er:YAG laser wavelength approximately 0.33 ms). Under these favorable circumstances, cooling of the tissue by heat dissipation is too slow resulting in the vaporization of cells during or immediately after application of the pulses of the second type.

In general, above the ablation threshold fluence Fₜ, the hottest part of the tissue close to the surface is ablated, in effect reducing the depth of the thermally affected skin layer. Ablation temperatures significantly above 100°C indicate that the water-absorbed laser ablation of human tissues is based on a micro-explosion mechanism. As the bound tissue water trapped within the microscopic cavities within the tissue absorbs the laser radiation, its temperature increases until the first boiling point (T ≈ 100 °C) is reached. Further heating converts one part of the water mass into vapor. As the vapor cannot expand freely, pressure in the cavity rises and exerts force on the cavity wall. Micro-explosive removal of tissue starts when the pressure exceeds the ultimate tensile strength of the tissue, which for soft tissue occurs at water vapor final "boiling" temperatures of T_{abl}≈ 255°C. Roughly speaking, for laser pulse fluences F above the ablation threshold the maximal temperature T stops increasing and remains at the ablation temperature T_{abl}.

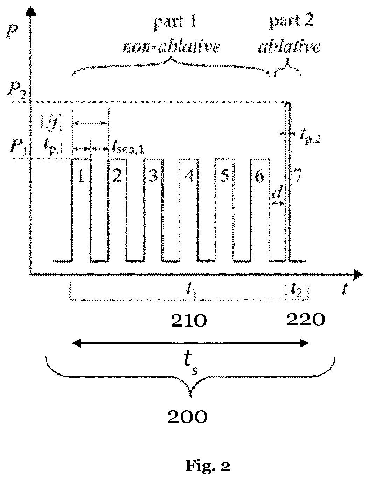

Fig. 2 represents another exemplary train of laser pulses 200. It comprises an exemplary number of N₁=6 laser pulses of a first type 210. In other examples, a smaller or larger number may be provided. All pulses of the first type may have the same shape and power level P₁ and they may collectively be referred to as part 1 of the laser pulse train. Generally, the labels used in Fig. 2 describe the same parameters as described with reference to Fig. 1. Notably, the first type of pulses 210 is non-ablative. That is, their peak power P₁, their duration t_{p,1}, their repetition rate and their total number are adapted to heat the tissue without ablating it. It is noted that the pulse period 1/f₁ may also be understood as the sum of the pulse duration t_{p,1} and the pulse separation t_{sep,1}.

The first type of laser pulses 210 may be followed, after a time d that may be selected as described herein, by a single ablative pulse of a second type 220. It may comprise a higher peak power P₂ than peak power P₁ of the first type of pulses. Additionally or alternatively, it may comprise a shorter pulse duration t_{p,2} than the pulse duration of the first type of pulses t_{p,1}. In other examples more than one ablative pulse of the second type may be provided, e.g. two. The second type of pulses 220 may collectively be referred to as part 2 of the laser pulse train.

In an embodiment according to Fig. 2, part 1 and part 2 of the pulse train may, e.g., be emitted from the same laser source for generating the at least one laser pulse. That is, they may for example comprise the same wavelength and/or they may be directed onto the tissue surface by the same applicator. This allows for seamless switch from a non-ablative to an ablative regime of the tissue without a need for calibration of two separate laser pulse modalities in-between. In one example, the same wavelength may be emitted during part 1 and part 2 of the pulse train by an Er:YAG laser source.

In another example, part 1 may be set up for heating of the tissue and be emitted by a deeper penetration laser, e.g. by an Nd:YAG source, and/or part 2 may be set up for ablation and be emitted by a water absorbed laser, e.g., by an Er:YAG laser source.

Since part 1 is non-ablative, e.g. by a suitable intensity or fluence per time, the temperature of a tissue surface irradiated by a laser pulse train comprising the at least one laser pulse may, e.g., be controlled by adjusting the laser parameters such as pulse duration, energy, pulse shape, pulse number N₁ and/or repetition rate. In order to heat the tissue surface to a maximum (peak) temperature T_{p,1-i}, e.g., of at least 70°C, 80°C, 90°C, 100°C, 125°C, 150°C, 175°C, 200°C, 225°C (below a tissue boiling temperature/ablation temperature that may be found at approximately 255°C), after each of the laser pulses, at least one laser parameter (e.g., pulse energy, pulse period, peak power, pulse diameter, number of pulses N₁ and/or pulse duration, etc.) may be carefully selected to ensure that i) a maximum peak temperature by the end of a pulse is below the critical temperature for tissue damage determined by the duration of the transient temperature spike; and ii) sufficient energy is delivered to the tissue surface, e.g. such that a certain base temperature is reached, without causing thermal damage (e.g. by keeping the base temperature T_{s,1} below 45° or below 65°), and at the same time remaining below ablation threshold. As for example outlined in European patent application EP 3 569 287 A1, which is incorporated herein by reference, short temperature spikes do not lead to thermal damage, providing that i) their maximal peak temperatures do not exceed the critical temperature for tissue damage, as determined by the thermal exposure time, and ii) as long as the longer-term baseline temperature is kept low enough.

The amount of energy delivered by each laser pulse and/or the time interval between each pulse in part 1 may be adjusted to control the temperature of the tissue surface. For example, when the laser pulse energy is increased, the tissue surface will absorb more energy, which will result in a stronger increase in temperature. Similarly, when the time interval between each pulse is decreased, the temperature of the tissue surface will increase as a result of the cumulative effect of the laser pulses.

The time interval d between part 1 and part 2 may also be adjustable or adjusted by a user of an apparatus described herein, e.g., the apparatus may comprise a corresponding user interface, such as a touch screen for adjusting d. In general, d may comprise values in the range of 1 ms to 500 ms, preferably 5 ms to 500 ms, or 5 ms to 100 ms. In other examples, the apparatus may comprise a number of predetermined modes which can be selected, e.g., in a menu displayed in the user interface, by the user, and which may comprise different values of *d* (and, e.g., different applicator modes). In one embodiment, following the end of non-ablative part 1, the time d may be set to allow optimal conditions for the superficial tissue ablation by part 2. For example, the pulses of the first type may be emitted with f₁ = 20 Hz repetition rate. Time d may be selected such that the pulse(s) of the second type follow with the same repetition rate, essentially emitting at least one ablative pulse (8th and 9^{th} pulse in Fig. 2, representing two pulses (j=1 and 2) of the second type).

The time durations *t*₁ of the first part, *t₂* of the second part and the duration *tₛ* of the total train comprising the first and second part may be dynamically adjustable or adjusted by user, e.g., the apparatus may comprise a corresponding user interface, such as a touch screen for adjusting *t*₁. It may also be set in regard to the pain threshold limit. The duration of *t*₁ may be determined in relation to the heat transfer mechanism that may include lateral (or transversal) and vertical (or longitudinal) heat diffusion processes, and thus, e.g., depend on the applicator mode.

The parameters of the pulses of part 1 may also be set to precondition for an optimal desired biological effect in part 2. For example, an additional pulse after part 1 may have a peak power of P = (P₁+P₂)/2 to additionally preheat the upper-most layer of the skin and allow for more effective soft tissue ablation in part 2.

In some examples, the energy delivery time t_{ed} of each laser pulse of the first type (and/or the second type) during which a second half of the respective laser pulse energy is delivered (t_{ed}; for temporally symmetric pulses this would be half of the pulse width) may be controlled so that, given a wavelength and thus a corresponding penetration depth of each laser pulse of the first type (and/or the second type, respectively) into the organic tissue, a thermal exposure time of the surface of the tissue of the patient is smaller than 900, smaller than 500, or smaller than 100 microseconds. They may be further determined by the criteria set out in European patent application EP 3 569 287 A1 which is fully incorporated by reference.

In some examples, the thermal exposure time may be understood as a time interval in which the temperature of the tissue surface is above Tₒ + (Tₘₐₓ - Tₒ )/2, wherein To defines an initial temperature of the tissue surface, and Tₘₐₓ defines the maximum peak temperature. Quantitatively, the thermal exposure time may be approximated by t_{ed} + (1/D) (δ + √(2Dt_{ed} ))² , wherein D = 0.1 mm² s⁻¹ is the thermal diffusivity of the treated tissue, as for example outlined in European patent application EP 3 569 287 A1, which is fully incorporated herein by reference.

The laser pulse of the second type may have a configuration to induce an ablative process within the tissue. Hence, it may have a fluence, e.g. for an Er:YAG laser, chosen to be at least 1.5 J/cm², for example, delivered with a pulse duration of 280 µs or less. Since the ablation threshold fluence increases when the pulse duration increases, the fluence of the pulse of the second type may also be adjusted according to pulse shape and/or pulse duration such that an ablation process is achieved.

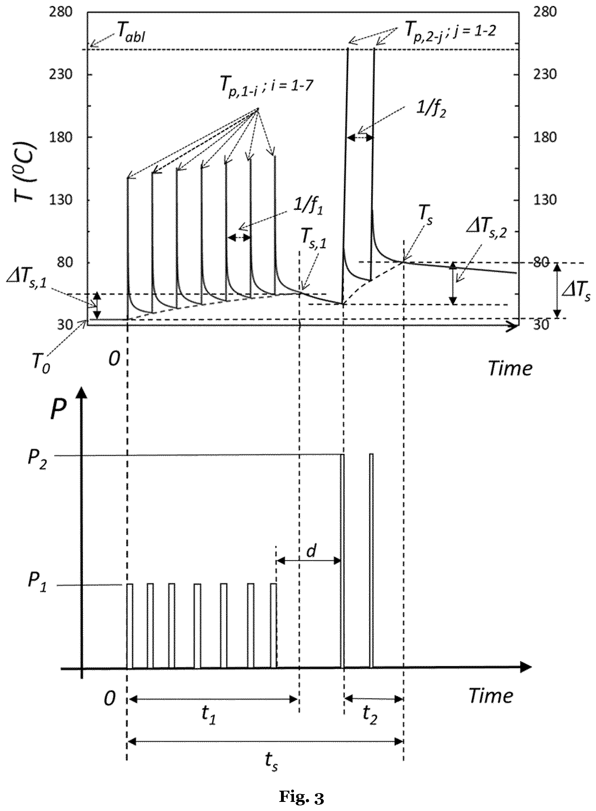

Fig. 3 shows another exemplary pulse train (lower part of Fig. 3) and a correspondingly achievable temperature profile in skin tissue (upper part of Fig. 3). In this example, pulses of the second type may not be delivered within a pulse period corresponding to that of the first type of pulses. Instead, the time d may be longer than the pulse period 1/f₁ (approximately equal to t_{sep,1} in Fig. 3) of part 1.

According to Fig. 3, similarly as in the example of Fig. 2, a first number N₁ of non-ablative pulses of the first type is delivered. In the example of Fig. 3, the first number is N₁ = 7, however also different numbers could be used. These pulses may each lead to a temperature increase/spike and a corresponding maximum temperature that stays below the ablation threshold temperature T_{abl} (about 255° C). However, due to the relatively quick succession of the pulses of the first type, the tissue cannot cool down completely in between pulses. Hence, although the sharp peak temperatures achieved by each pulse decay significantly until the respective next pulse is applied, they do not decay completely, such that there is a build-up of the baseline temperature by ΔT. That is the initial temperature is elevated by ΔT_{s,1} to T_{s,1}. This temperature may be defined at time t₁ = N₁/f₁ after the start of the first pulse, i.e., a time 1/f₁ after the start of the last pulse of the first type.

At a time d after the temperature has started to decay from T_{s,1}, additional two above-ablation-threshold pulses of a second type may be applied. These pulses may comprise a higher peak power P₂ compared to the peak power P₁ of the first pulses. It is noted that the pulse duration of the second type of pulses may differ from the pulse duration of the pulses of the first type or be identical. In the example of Fig. 3, the duration of the second type of pulses is shorter than the pulse duration of the pulses of the first type. The higher peak power may be adapted to lead to ablation, i.e., a temperature peak at or above T_{abl}.

The temperature within the organic tissue induced by the pulses, as shown in Fig. 3, behaves as follows. From an initial temperature of about 35°C, every one of the first 7 laser pulses heats the temperature up to more than 140 °C, preferably up to less than 180°. As the laser pulses are of very short pulse duration, the surface temperature cools down quickly below the target temperature T_{s,1}. T_{s,1} may be approximately 55 °C. Before the temperature can relax to its initial value after the first laser pulse, another laser pulse takes back the temperature to over 140°C, this time slightly higher than the first time. Yet again, the temperature cools down below T_{s,1}. Before the initial temperature is reached, the third laser pulse hits the tissue surface leading to another temperature peak. This way, the regular laser pulses cause succeeding heat spikes of the tissue surface, each of these heat spikes being higher than the preceding heat spikes. None of these heat spikes, however, exceeds the ablation threshold T_{abl} above 255°C. This succession of heat spikes causes a continuous raise of the overall surface temperature of the organic tissue, eventually raising the temperature of the tissue to the target temperature T_{s,1}.

In some examples, the first ablative pulse of the second type may be applied at a time (and d may be set accordingly), at which the temperature has decreased from T_{s,1} by Δ'T_{s,1} to about T_{s,1} - ΔT_{s,1}/₃. The laser pulse of the second type is thus emitted when the surface temperature has cooled down by ΔT_{s,1}/₃. In other examples, d can also be for example be set such that the pulse of the second type is applied when Tₛ drops for 1/5 of the initial ΔT or for ¼, ¾, etc. The respective times may be measured experimentally, e.g., for a corresponding illumination pattern, and the apparatus may then be programmed accordingly, e.g. using a look-up table. That is, the apparatus may comprise a user interface, in which the user can select d and/or the desired temperature drop as outlined above. As it takes longer to cool down more, the corresponding time delays d are longer for higher temperature decreases, and vice versa, than in the first case.

As shown in Fig.3, the ablative pulses of the second type also contribute to the increase of the baseline temperature, in the amount of ΔT_{s,2}. The final (peak) baseline temperature increase is then equal to ΔTₛ = ΔT_{s,1} - Δ'T_{s,1} + ΔT_{s,2}.

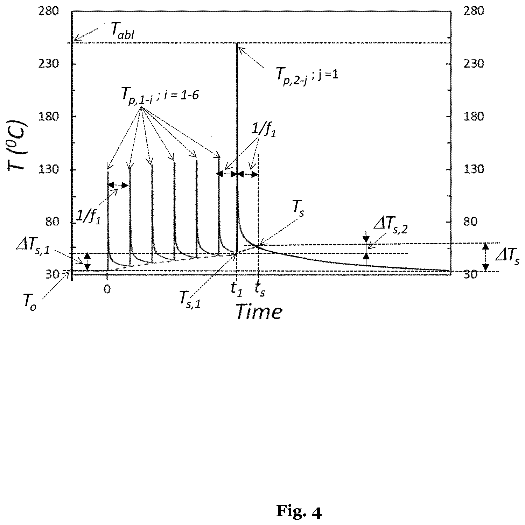

Fig. 4 shows the surface temperature of an organic tissue during an exposure to a train of seven laser pulses all seven of them separated by the same time period equal to 1/f₁. From this train, the first N₁ = 6 pulses are laser pulses of the first type (i.e. the fluences Fᵢ of these pulses are below the ablation threshold of the organic tissue Fₜ, including when taking into account that Fₜ gets reduced due to the slow rise of the base temperature). In some examples, the energy delivery time t_{ed} of each laser pulse of the first type during which a second half of the respective laser pulse energy is delivered (t_{ed}; here: half of the pulse width) may be controlled so that, given a wavelength and thus a corresponding penetration depth of each laser pulse of the first type into the organic tissue, a thermal exposure time of the surface of the tissue of the patient is smaller than 900, smaller than 500, or smaller than 100 microseconds. They may be further determined by the criteria set out in European patent application EP 3 569 287 A1 which is fully incorporated by reference.

At least one pulse of the second type, an ablative pulse (j=1) is added to the plurality of laser pulses of the first type. This laser pulse may have a fluence Fⱼ ≥ Fₜ, wherein Fₜ is the fluence corresponding to the ablation threshold.

Starting from a surface temperature of *T*₀ = 35°*C*, every laser pulse induces temperature spikes on the tissue surface, however, as in the case of Fig. 3, the peak temperatures resulting from the first laser pulses are respectively below the boiling temperature/ablation temperature of the tissue *T_{b}* ≈ 255°*C*. Comparable to Fig. 3, the baseline temperature rises continuously. This time, however, the laser pulse of the second type is applied shortly after the last pulse of the first type. In fact, the first laser pulse of the second type may be applied at time d = 1/f₁ after the last laser pulse of the first type, as explained with reference to Fig. 2. The six laser pulses of the first type increase the baseline temperature by ΔT_{s,1}. Since the first pulse of the second type is delivered at d = 1/f₁, there is no baseline temperature decrease before the ablative pulse of the second ty pe is delivered (i.e., Δ'T_{s,1} = 0). The laser pulse (j =1) of the second type first transiently heats the surface of the organic tissue up to about the ablation temperature T_{abl} resulting in the tissue's ablation. Due to this first (and optionally single) laser pulse of the second type, the tissue baseline temperature further rises by ΔT_{s,2}, resulting in the final (peak) baseline temperature of Tₛ = Tₒ + ΔTₛ = Tₒ + ΔT_{s,1} + ΔT_{s,2}.

Surprisingly, this configuration was found to reduce patient's pain during a treatment significantly compared to a treatment comprising seven instead of six pulses of the first type and no ablative pulse of the second type. When the cumulative fluence of the seven pulses of the first type was adjusted to result in the same final temperature as obtained with the pulse sequence shown in Fig. 4, i.e., when T_{s,1} was equal to Tₛ, it was determined via various experiments using a full beam handpiece that the obtained pain score increased by 1.8 points. The above pain score refers to the standard VAS ("Visual Analogue Score") method, which is a validated, subjective measure for pain intensity from 1-10. Similarly, it was shown that with the additional ablative pulse a final painless temperature of Tₛ = 56°C could be attained which is substantially above the pain threshold for treatments without anaesthesia of 47 °C, demonstrating the efficacy of the additional ablative pulse.

The observation may be explained by gate control theory of pain. The gate control theory of pain is a mechanism, in the spinal cord, in which pain signals can be sent up to the brain to be processed to accentuate the possible perceived pain, or attenuate it at the spinal cord itself. The "gate" is the mechanism where pain signals can be let through or restricted. Different kinds of nerve fibers are responsible for carrying different kinds of messages. Large nerve fibers send the brain information about, for example, being touched or feeling pressure on the skin. Small nerve fibers send the brain information about pain and temperature. Large fiber activity sends messages to the brain very quickly, while small fiber activity moves more slowly. According to gate control theory, the sensation of noxious stimuli can be blocked by non-noxious stimuli carried by nerve fibers that reach the brain before the painful input because those nerve fibers are slower. Studies show that painful stimuli are blocked from getting to the brain by fibers that transmit their sensation faster. Stimulating large fiber activity, can therefore help "close the gates" by getting to the brain first, and not letting pain signals (carried by small fibers) to proceed to the brain.

The painful noxious stimulus carried by small fibers is represented by the increase ΔTₛ of the skin temperature. The additionally applied non-noxious signal carried by large fibers may be represented by the sound and the tactile skin sensation of the recoil force when the laser-ablated material escapes from the surface with high velocities up to the speed of sound. According to tests performed with an Er:YAG laser, the gate mechanism may be activated at very low ablation conditions, at fluences as low as F₂₋ⱼ = 1.2-10 J/cm², where the depth of ablation d_{abl} is in a range of about 5-50 µm. A pulse with this ablation depth may be considered as a relatively non-harmful stimulus. The maximal depth of ablation may be less than about 50 microns, which is still in epidermis. Thus, the pulses may be adapted such that there is no damage to vessels in the lower lying dermis, which may cause bleeding. So, the application of the pulses may be blood-free. No coagulative measures are therefore needed or otherwise performed to stop any bleeding.

In certain examples, it may be desirable that the non-noxious signal generated by the ablative pulse does not only block the pain signal resulting from the final temperature increase ΔTₛ but also the pain signal resulting from the baseline temperature increase ΔT_{s,1} following the preceding non-ablative sequence. In this case, the delay d between the last pulse of the first type of laser pulses and the first ablative pulse j =1 may be chosen to be shorter than the time required for the noxious signal to reach the brain, therefore preferably shorter than 450 ms, more preferably shorter than 300 ms, most preferably shorter than 100 ms.

It is important to note that the additional ablative pulse j may not only be considered to reduce pain, but at the same time also contributes to the increase of the baseline temperature Tₛ (described by temperature rise ΔT_{s,2}). It is therefore important to limit the total delivered fluence ΣFᵢ + ΣFⱼ to values where the temperature Tₛ is not only below the gate-increased pain threshold but also below the tissue's individual threshold beyond which irreversible damage would occur.

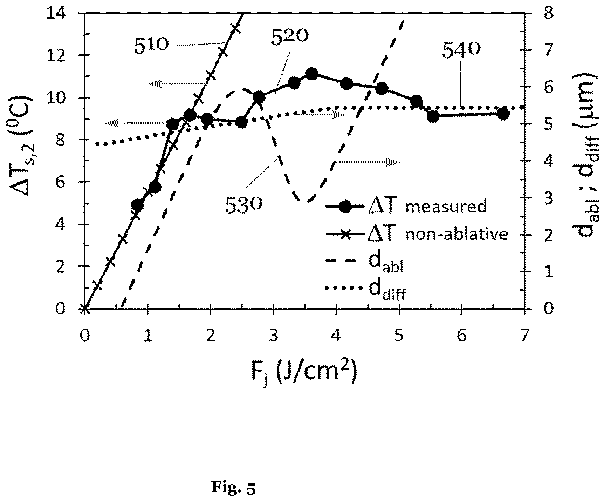

Fig. 5 refers to the situation of Fig. 4 where one (ablative) pulse of the second type follows a plurality of (non-ablative) pulses of the first type. Fig. 5 examines the temperature rise ΔT_{s,2} of the temperature of the organic tissue due to the energy delivered by the one pulse j of the second type depending on the fluence F₂₋ⱼ of this pulse.

The depicted dependence was obtained by thermal camera measurements on a hand palm skin with a modified Fotona TimeWalker^{®} Er:YAG laser device using a full beam Fotona R11 handpiece (set to d' = 3 mm). The device was adapted to deliver a first and a second part within a sequence of 7 pulses, separated by 1/f₁ = 50 msec, resulting in tₛ = 350 ms. The first six pulses representing the first (non-ablative) part with duration t₁ = 300 ms, had the same fluence Fᵢ and the same nominal duration t_{p,1} ≈ 0.5-0.6 ms. The pulse fluence was adjusted to result in a cumulative fluence F₁ = ΣFᵢ (i =1-6) = 1.7 J/cm². This ensured that the fluence Fᵢ = 0.28 J/cm² of individual pulses was below the skin ablation threshold. A seventh pulse representing the second (ablative) part had a nominal duration of t_{p,2} ≈ 0.25 ms, with the actual measured laser pulse duration increasing from t_{p,2} ≈ 0.18 msec at low fluences to t_{p,2} ≈ 0.27 msec at fluences above about 4 J/cm². The peak baseline temperature increases ΔT_{s,1} and ΔT_{s,2} generated by respectively the six pulses (i = 1-6) of the first type, and the single pulse (j =1) of the second type, were measured using a thermal camera for different values of the fluence of the single ablative pulse of the second type, Fⱼ (j =1) over the range of Fⱼ = 0.8 - 6.7 J/cm².

As outlined above, the baseline temperature increase ΔT_{s,1} is expected to depend on the cumulative fluence F₁ as ΔT_{s,1} = F₁ x (Aₛ x t₁ ^{-Ks}). It is also to be appreciated that for fluences Fⱼ below ablation threshold, the temperature behavior should be the same as expected from seven non-ablative pulses of the first type according to ΔTₛ = (F₁ + Fⱼ) x (Aₛ x tₛ ^{-Ks}), resulting in the baseline temperature increase for fluences Fⱼ below threshold of ${ΔT}_{s , 2} = {F}_{j} x \left({A}_{s} x {{t}_{s}}^{- Ks}\right) = {F}_{j} x {L}_{R 11}$ where L_{R11} represents the full beam temperature coefficient.

In Fig. 5, this dependence is presented by Graph 510. As outlined, for a full beam irradiation, the parameter Aₛ has been determined to be Aₛ = (84 ± 10) °C cm²/J for treatments on skin and Aₛ = (64 ± 10) °C cm²/J for treatments on human mucosa. Similarly, the parameter Kₛ has been found for both tissues to be equal to Kₛ = -(0.43± 0.05) when tₛ is expressed in milliseconds (msec). Thus, L_{R11} should be equal to 6.7 (1 ± 12%) °C cm²/J. As shown by Graph 510, the actually measured full beam coefficient is equal to approximately 6 °C cm²/J, which is within the expected range of the coefficient. The slightly smaller value of the coefficient than average according to the model can be attributed to the remaining small influence of the transverse heat diffusion due to the small spot size (d' = 3 mm), contributing slightly to the predominant longitudinal tissue cooling, and thus effectively reducing the final temperature.

Graph 520 shows the real temperature evolution obtained by the above-described method. It may be observed that graphs 510 and 520 are substantially aligned for small fluences Fj ≤ 1 J/cm². However, the temperature essentially stops increasing for fluences Fⱼ above 1.7 J/cm², representing the ablation threshold Fₜ. Roughly speaking, based on the temperature measurements under full beam conditions, regardless of the actual higher pulse fluence, each ablative pulse of the second type contributes about F_{j-eff}= Fₜ ≈ 1.7 J/cm² to the overall temperature increase. The difference is used up for ablation.

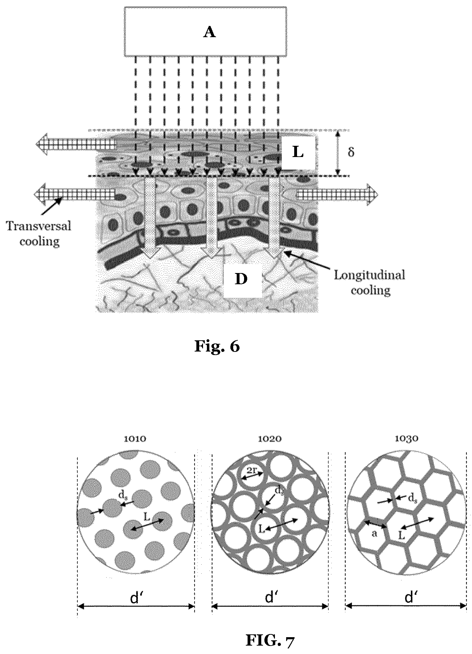

The above observation applies generally, for any number of pulses N₁ and N₂, and not just for a specific case of N₁ = 6 and N₂ = 1 presented in Fig. 6. For the full beam configuration, the final baseline temperature increase following the pulse trains of the first and second type can be roughly estimated from ${ΔT}_{s} = \left({F}_{1}+{N}_{2} x {F}_{j - eff}\right) x \left({A}_{s} x {{t}_{s}}^{- Ks}\right)$ where the effective fluence F_{j-eff} is equal to the ablation threshold fluence, characteristic for a particular combination of the tissue properties and laser parameters. Measured threshold fluences for water-absorbed wavelengths in skin and mucosa for pulse durations t_{p,2}≤ 1 ms vary between 0.5 J/cm² and 10 J/cm².

Looking more closely, while the temperature increase is expected to remain constant after reaching the "boiling" point, the temperature is in Fig. 6 observed to continue to vary by passing through several ablation regimes, until it settles at a final steady state temperature increase (equal to about 9 °C for the case presented in Fig. 6). Therefore, Fig. 6 allows for identifying preferred parameters for ablation characterized by small ΔT_{s,2}.

The observed behavior can be explained by noting that very roughly, the water in body tissue is present in two forms, as free and bound water. Free water is a loosely bound form of water that lies in the space between and around bodily tissue, while bound water cannot be easily extracted from the tissue. It is the changes in the water content during ablation that influence the relationship between the ablation depth and the longitudinal heat diffusion, and subsequently the final baseline temperature Tₛ.

Graph 530 represented by a dashed line shows a model for the development of ablation depth *d_{abl}.* As pointed out above, an ablation depth is understood to be the depth of material removed per laser pulse. In many systems, *d_{abl}* may be expressed by *d_{abl} =* $δln \left(\frac{{F}_{j}}{{F}_{t}}\right)$ where δ is the optical penetration depth of the organic tissue. For the purpose of this invention, the ablation depth may be estimated by d_{abl} ≈ (Fⱼ -Fₜ)/hₐ where hₐ is the specific ablation energy (J/mm³) of the organic tissue. This approach exploits the relationship Fₜ = hₐδ and a first order Taylor-development for Fⱼ close to Fₜ.

The model further assumes that, due to increasing desiccation of the hydration level of the organic tissue (in this case the uppermost skin layer called stratum corneum) and a consequent reduced light absorption by the remaining water, the ablation threshold undergoes a transition from 0.6 J/cm² to 2.85 J/cm². These values are obtained from hydration level estimations of initially 38% and finally 8%. That is, the ablation depth would be expected to increase with increasing fluence of the single pulse. However, as the fluence increases, there is an increased desiccation, leading to less absorption and thus less ablation. Hence, the ablation depth may stagnate or even get shallower with increasing fluence (cf. range from 2 to 3.4 J/cm² in Fig. 6). At some point, the tissue will be fully desiccated, at which the ablation depth will again increase with increasing fluence (cf. range > 3.5 J/cm² in Fig. 6).

Roughly speaking., below Fₜ there is no ablation, while above Fₜ the ablation depth d_{abl} increases linearly with fluence. The specific heat of ablation (hₐ) depends primarily on the water content H and laser wavelength. For the water-absorbed lasers, hₐ varies between 1 and 4 J/mm³. Ablation depth thus depends on the ablative fluence F-Ft, as d_{abl} ≈ K x (F-Fₜ), where K ≈ 2-5 µm/(J/cm²). For the Er:YAG laser wavelength and t_{p,2} = 0.3 ms, our measurements on skin show that K = (3.7 ± 0.3) µm cm²/J.

Graph 540 represented by the dotted line shows the development of a modelled diffusion depth ${d}_{diff , long} = 2 \sqrt{{Dt}_{p}}$*.* The slight increase of the diffusion depth with fluence is due to the gradual increase of the laser pulse duration t_{p,2} with laser fluence Fⱼ.

The first regime is the warm ablation regime I centered around 1.4-1.9 J/cm². In this regime, the ablation depth d_{abl} is smaller than the longitudinal diffusion length d_{long} . This means that ablation speed is not high enough to catch-up with heat diffusion, and therefore a thin heated layer remains at the surface also by the end of the pulse. This results in a "warm" ablation regime where ΔTₛ is higher than it what would be if all seven pulses were sub-ablative.

The first cold ablation regime II (2.0 - 2.6 J/cm²) represents the ablation regime where with increased fluence Fⱼ the ablation speed becomes faster than heat diffusion, resulting in reduced ΔT_{s,2}. In this regime, the stratum corneum's water content is expected to still remain at the initial level. The resulting ablation depth d_{abl} in this regime is relatively high, effectively ablating away the longitudinal diffusion-heated tissue layer.

The hot ablation regime III (2.6 - 5 J/cm²) represents the ablation regime where the freely bound water gets evaporated, reducing the water content to below H = 10%. Consequently, the optical absorption depth dₒₚₜ, laser threshold fluence Fₜ and specific heat capacity hₐ also undergo a transition. As a result, the ablation rate becomes again slower than heat diffusion, leaving a hot tissue layer by the end of the laser pulse.

Finally, the second cold ablation regime IV (Fⱼ > 5 J/cm²) represents the ablation regime where the ablation speed of the desiccated tissue becomes at higher fluences again faster than heat diffusion.

As is described hereabove, limiting the temperature within the organic tissue is important for both pain reduction and prevention of irreparable or undesired tissue damage. From Fig. 6, it may thus be inferred that it is advantageous to work in the cold ablation regimes with fluences of about 2 to 2.6 J/cm² and above about 5 J/cm². In this range, *d_{diff}* ≤ *d_{abl}* holds. In some examples, the ablation depth is larger than 5 µm or larger than 6 µm, and the diffusion depth is below 5 µm or below 6 µm. In this situation, the energy delivered by the laser pulse of the second type does not contribute to the heating of the organic tissue, and there is cold ablation.

It is to be noted that this (first) "cold" regime as shown in Fig. 6 is present only for Er:YAG pulse durations t_{p,2} for which the thermal diffusion depth d_{diff} is smaller than the ablation depth d_{abl} ≈ 6 µm just before the desiccation becomes appreciable $\left({d}_{diff}=\sqrt{{Dt}_{p}}\right.$ depends essentially on pulse duration tₚ). Therefore, for Er:YAG pulse durations longer than about 0.33 ms, as the fluence is increased, the ablation proceeds directly from the warm to the hot regime without passing through the first cold regime (until it reaches the conditions for second cold ablation at higher fluences).

It is noted that under the preceding condition, the ablation is somehow self-regulatory. With the ablation process, a part of the superficial tissue is removed, thereby also removing the volume of tissue that absorbed the highest amount of energy (e.g., has the highest temperature). This increases safety.

Another condition is that the ablative pulse should be only superficially ablative to reduce pain, and therefore the ablation depth should not be larger than d_{abl-max} being equal to about 50 µm, preferably equal to 30 µm, most preferably equal to 10 µm.

Fig. 6 shows a schematic view of dynamic heat distribution processes. A (laser) light source A emits light onto an organic tissue such as the stratum corneum or mucosa. The heated surface will yield energy to deeper layer and adjacent areas within the tissue according to the temperature gradient. In general, deeper layers are characterized by a strong gradient, so longitudinal diffusion is encouraged. This has a cooling effect on the surface, however, diffusion to deeper tissue layers may cause damages or pain. It is therefore advantageous to expedite transversal cooling, too. However, if a surface is fully covered by a light beam, there will not be any substantial transversal heat gradients within the illuminated area. Thus, no inner cooling can take place (of course, outer transversal cooling may take place, i.e. transversal cooling from the borders of the illuminated area). To reduce longitudinal heat gradients, it is advantageous to enable transversal cooling within the illuminated area. This may be done by illuminating a tissue surface area according to a pattern.

Fig. 7 shows various exemplary illumination patterns the first and/or second type of pulses may have (cf. EP 4 046 585 A1 and US 12,226,152 B1).

On the left side, an illumination pattern 1010 comprising a polka dot lattice of laser spots is shown. The laser spots have a diameter of dₛ and their centres are respectively spaced by L. The total area encircled by the outmost laser illumination contour may be circular and may have a diameter of 5 to 50 mm, for example.

In the centre, an illumination pattern 1020 comprising a ring lattice is shown. Each ring is in connection with 6 other rings and/or has 6 other rings as next neighbours. The line width of each ring is denoted by dₛ. The centre to centre spacing is represented by L and the inner diameter of every ring is 2r.

On the right side, an illumination pattern 1030 comprising a honeycomb lattice is shown. It is constituted by interconnected regular hexagons. Each hexagon centre is spaced by L from an adjacent hexagon centre. The line width is denoted dₛ and the side length of the hexagons is marked as a.

The time t₁ may in some examples be configured to best match the thermal relaxation time of the illumination pattern used for the first type of pulses and/or the desired depth of the thermal effect. Based upon the desired depth of a thermal effect *z*ₜₕ, a time duration t₁ of part 1 of the laser pulse train can be estimated. For example, it can be estimated as t₁= (*z*ₜₕ - δ)² / (4D), where D is thermal diffusivity of the tissue. Thus, a longer duration t₁ generally allows achieving deeper tissue regions. A user interface of the apparatus may be configured to display and/or allow selecting a certain diffusion depth zₜₕ, and the apparatus may adapt the parameters of the corresponding type of pulses accordingly, e.g., based on the above formula and/or one or more look-up tables. The apparatus may comprise at least two different modes, which may be selected by the user, e.g., via a user interface, in which two different diffusion depths are set. In other examples, the at least two different modes may be adapted to provide the same diffusion depths using different illumination patterns.

The time needed for a certain thermal effect may also depend on the illumination pattern and should therefore be adjusted accordingly. For example, if the laser beam illuminates 100 % of the irradiated area, the temperature field is relatively homogeneous. The temperature gradient in transversal direction in homogeneous temperature fields is essentially non-existent, hence the heat diffusion in transversal direction is negligible and longitudinal heat diffusion dominates the temperature distribution process. However, if the laser beam has a patterned illumination essentially covering less than 100 %, a higher ratio between transversal and longitudinal heat diffusion increases the cooldown in transversal direction, essentially resulting in more superficial thermal effect *z*ₜₕ.

Hence, t₁ may be varied depending on illumination patterns. For example, if the surface coverage of a first illumination pattern is less than 50%, the duration t₁ may be increased by a first duration, compared to durations used for illumination patterns having a surface coverage of more than 50%. Similarly, an additional increase by a second duration may be applied for a second illumination pattern that has a surface coverage of less than 30%. Thus, different modes comprising different illumination patterns may be adapted to provide a targeted diffusion depth, e.g. a same diffusion depth.

This may be particularly useful when using laser sources that are highly absorbed within a thin layer of superficial tissue, yielding a very distinctive thermal pattern with high thermal gradients between illuminated and non-illuminated areas, and where thermal diffusion greatly impacts the thermal relaxation times.

The following Table lists exemplary transverse thermal relaxation times for various illumination patterns, such as polka dots as shown at reference sign 1010 of Fig. 7, circular rings as shown at reference sign 1020 of Fig. 7, hexagonal lines arranged in a honeycomb (HC) lattice, similarly as shown at reference sign 1030 of Fig. 7, compared with a single full spot with 100% surface coverage:

**Table 1**

| | | **Polka dot** | **Rings** | **Honey comb** | **Full spot** |
|---|---|---|---|---|---|
| **dₛ (mm)** | | 0.5 to 2 | 0.13 | 0.16 | 7 |
| **N spots** | | 15 to 25 | 19 | 19 | 1 |
| **r or a (mm)** | | | 0.87 | 1.15 | |
| **S (mm²)** | | 0.5 to 2 | 0.76 | 1.05 | 38.465 |
| **Sₜₒₜ (mm²)** | | 7.5 to 50 | 14.50 | 9.99 | 38.465 |
| **Coverage (%)** | | 30 to 50% | 30 to 50% | 15 to 35 % | 100% |
| **Transverse TRT (s)** | | 0.5 to 0.7 | 0.5 to 0.7 | 0.15 to 0.35 | 25 to 35 |

The spot size d' and their distance x may be chosen such that the spot size d is in the range of 0.3 mm ≤ d ≤ 1.5 mm, and that the tissue coverage TC = (N x area(S))/area(Sₜₒₜ) (in %) is in the range of 25% ≤ TC ≤ 65 %. Further, the size (e.g., diameter) of the treatment area Sₜₒₜ which comprise all the spots is in the range of 3 to 30 mm. These parameters may ensure that, during the time span between two pulses, e.g. of the first type, the thermal diffusion in the lateral direction spreads the heat which is generated by the laser radiation away from a localized spot S towards the surroundings of the spot, thus effectively spatially homogenizing the slowly varying temperature across the area Stot. Thus, intense heat shocks with short exposure time can be delivered to the localized spots S, without causing long-term overheating of the treatment area. This way, it is possible to create an intense heat "needling" on about 50% of the surface of the tissue, whereas the thermal injury in deeper layers is approximately homogeneous (in the lateral direction), since, in the deeper layers, the thermal diffusion spreads the heat in all directions.

In other examples, the precise geometry of the illumination patterns may differ from those indicated in Table 1. Importantly, using the patterned illumination, a significant surface coverage can be achieved (in some examples: 39%, 38%, and 26%), but still a reduction of the transverse TRT by two orders of magnitude may be achieved (from for example, 27.84 seconds, to 0.57 s, 0.61 s, and 0.23 s, respectively).

For example, the apparatus may allow user selection of a certain illumination pattern for the pulses of the first type (e.g. out of the mentioned options), and then the time d between first and second types of pulses may be automatically adjusted by the apparatus, such that a certain thermal effect may be achieved by the first type of pulses (e.g., as preset for a certain predetermined treatment mode of the apparatus, or as specifically selected by the user).

The polka dot lattice represents an example of such patterned illumination wherein the laser beam irradiates a number of individual spots within the treatment area S', each spot having the size (e.g., diameter) *d*ₛ is separated from a neighboring spot by the distance **L.**

The spot size *d*ₛ and the distance L are chosen such that the spot size *d*ₛ is in the range of 0.3 mm ≤ *d*ₛ ≤ 1.5 mm, and that the tissue coverage TC is in the range of 25% ≤ TC ≤ 65 %. Further, the size (e.g., diameter) of the treatment area S' which comprise all the spots is in the range of d' = 3 to 30 mm. These parameters ensure that, during the time span between two pulses, the thermal diffusion in the lateral direction spreads the heat which is generated by the laser radiation away from a localized spot towards the surroundings of the spot, thus effectively spatially homogenizing the slowly varying temperature T_{s,1} across the area S'. Thus, intense heat shocks with short exposure time can be delivered to the localized spots, without causing long-term overheating of the treatment area S'. This way, it is possible to create an intense heat "needling" on about 50% of the surface of the tissue, whereas the thermal injury in deeper layers is approximately homogeneous (in the lateral direction), since, in the deeper layers, the thermal diffusion spreads the heat in all directions. For a patterned beam irradiation with the fluence F₁ representing the cumulatively delivered fluence F₁ = ΣFᵢ within the spots, the parameter Aₛ is reduced approximately by a pattern factor TC. This reduction of the final target temperature is due to the fact that during the relatively long sequence duration of the pulses, the heat generated within the spots has sufficient time to diffuse not only deeper into the tissue but also radially away from the spots, effectively spreading the temperature over the whole treatment area S', with the final baseline temperature being homogeneous over the whole treatment area S'.

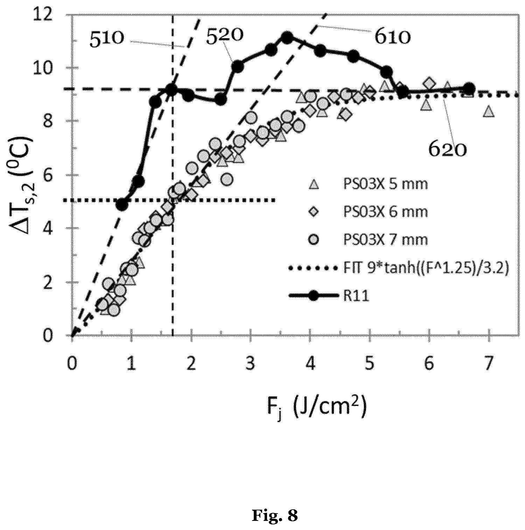

It is important to note that using a patterned beam does not influence only the tissue temperature behavior during the first, non-ablative part but also the temperatures during the second, ablative part. This can be seen from Fig. 8 which shows the temperature elevation depending on the fluence Fⱼ of a single pulse of the second type as has been exhibited in Fig. 5. However, additionally to the temperature measurement resulting from the use of a full laser beam (in Fig. 8 referred to as "R11"), patterned laser beams in the shape of polka dots were used, too. Measurements for overall spot diameters of d' = 5, 6 and 7 mm, with the micro spot diameters of about dₛ ≈ 1 mm and L ≈ 3dₛ/2, resulting in tissue coverage of about TC ≈ 30% were carried out (referred to as PS03X 5,6 and 7 mm).

Similarly to the full beam configuration, the baseline temperature increase ΔT_{s,1} for the patterned beam configuration is expected to depend on the cumulative fluence F₁ as ΔT_{s,1} = F₁ x (Aₛ x t₁ ^{-Ks}). It is also to be appreciated that for fluences Fⱼ below ablation threshold, the temperature behavior should be the same as expected from seven non-ablative pulses of the first type according to ΔTₛ = (F₁ + Fⱼ) x (Aₛ x tₛ ^{-Ks}), resulting in the baseline temperature increase for fluences Fⱼ below threshold of ${ΔT}_{s , 2} = {F}_{j} \times \left({A}_{s}\times {{t}_{s}}^{- Ks}\right) = {F}_{j} \times {L}_{PSo 3 x}$ where L_{PS03} represents the patterned beam temperature coefficient. This dependence is in Fig. 8 presented by Graph 610. Roughly, the patterned beam coefficient can be estimated from the full beam coefficient according to L_{PS03} ≈ NC x L_{R11} where L_{R11} = 6.7 °C cm²/J is the full beam coefficient for spotsizes d' = 5-7 mm. The measured patterned beam coefficient is equal to 2.8 °C cm²/J , which is larger than L_{R11/}3 , as would be expected from the TC = 30% coverage. This can be explained by the fact that the thermal camera detected the maximal temperature within the polka dot, and that by the time tₛ = 350 ms when the temperature measurement was taken the transverse heat diffusion has not had enough time to spread the heat homogeneously over the overall spot S'.

A surprising observation is that the actually measured temperatures represented by light symbols in Fig. 8 do not stop increasing at the boiling temperature reached at Fⱼ = F₁ ≈ 1.7 J/cm² which would correspond to ΔT_{s,2} ≈ 5 °C, but continues to grow until the temperature saturates approximately at the same temperature increase ΔT_{s,2}≈ 9 °C, as was the case for the full beam measurement. As shown by Graph 620, the temperature increase can be approximated by ${ΔT}_{s , 2} = {C}_{o} \times tanh \left({{F}_{j}}^{{C}_{1}}/{C}_{2}\right)$ where C₀ = 9 °C, C₁= 1.26 and C₂ =3.2, with Fⱼ in units of J/cm².

This observation can be explained by the fact that the amount of the transverse heat transfer depends on the time during which the polka dot spot is at the ablation temperature. At very high fluences, the polka dot heat source is at the ablation temperature practically over the whole duration of the laser pulse. The delivered energy of the diffusion heat source was therefore proportional to the square area = t_{p,2} x T_{abl}. On the other hand, at the threshold fluence the polka dot heat source is at the ablation temperature only for a short moment by the end of a laser pulse. The delivered energy of the diffusion heat source in this case was therefore proportional to the triangular area = t_{p,2} x T_{abl} /2. This may explain why the final temperature rise at threshold is about one half of the final temperature rise ΔT_{s,2} at high fluences Fⱼ.

Another observation is that with the patterned beam, the influence of ablation regimes I - IV on ΔT_{s,2} is less pronounced than with the full beam. This is because the ablation regimes are governed by the effects of the longitudinal heat diffusion. These effects are dominant for the full beam but are reduced proportionally to the coverage ratio (in this case by TC =30%) of the patterned beam. This represents another important advantage of using a patterned beam since any fluence above ablation threshold can be used without paying attention to which ablation regime, cold, warm or hot, the selected fluence corresponds to.

Thus, using the concepts described herein with patterned illumination provides for a particular synergy.

As is the case for the full beam configuration, the above observation applies generally, for any number of pulses N₁ and N₂, and not just for a specific case of N₁ = 6 and N₂ = 1 presented in Fig. 8. For the patterned beam configuration, the final baseline temperature increase following the pulse trains of the first and second type can be roughly estimated from ${ΔT}_{s} \approx \left({F}_{1}+{N}_{2}\times {F}_{j - eff}\right) \times \left({A}_{s}\times {{t}_{s}}^{- Ks}\right)$ where the effective fluence F_{j-eff} ≈ Fⱼ for Fₜ< Fⱼ ≤ 2Ft, and F_{j-eff} ≈ 2Ft for Fⱼ> 2Fₜ.

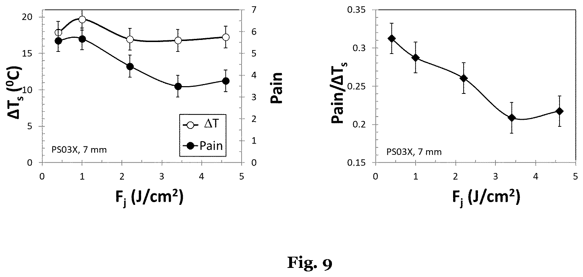

Fig. 9 shows the above-mentioned pain reduction effect in the case of a patterned laser beam (polka dot laser beam (PS03X) having a spot diameter d' of 7 mm). As is the case shown in Fig. 8, the Fotona TimeWalker Er:YAG laser device was adapted to deliver a first and a second part within a sequence of 7 pulses, separated by 1/f₁ = 50 msec, resulting in tₛ = 350 ms. The first six pulses representing the first (non-ablative) part with duration t₁ = 300 ms, had the same fluence Fᵢ and the same nominal duration t_{p,1} ≈ 0.5-0.6 ms. The pulse fluence was adjusted to result in a cumulative fluence F₁ = ΣFᵢ (i =1-6) = 2.6-5.5 J/cm². A seventh pulse representing the second (ablative) part had a nominal duration of t_{p,2} ≈ 0.25 ms, with the actual measured laser pulse duration increasing from t_{p,2} ≈ 0.18 msec to at low fluences to t_{p,2} ≈ 0.27 msec at fluences above about 4 J/cm². The temperature increase and pain level are shown, respectively, as a function of the fluence of the single pulse of the second type Fⱼ.

Different combinations of the cumulative fluence Fₛ of the pulses of the first type of pulses and the fluence Fⱼ of the pulse of the second type (ablative) with the total delivered fluence F'ₛ = Fₛ + Fⱼ were selected to correspond to approximately the same final temperatures. The actual realized temperatures were measured by a thermal camera. Pain was evaluated using the mentioned standard VAS method. Measurements were made on 5 patients, and averages were made.

The diagram on the left of Fig. 9 shows the overall temperature elevation (ΔTₛ) and the subjective pain score VAS, depending on the fluence Fⱼ attributed to the last (ablative) pulse of the second type. As can be seen, for higher fluences Fⱼ, the subjective pain shows a strong reduction, e.g., from initially above 5 (fluence Fⱼ < 1 J/cm²) to below 4 (fluences > 3 J/cm²). Correspondingly, the ratio of pain per temperature elevation is depicted in the diagram to the right, showing a substantial reduction of this value from above 0.3/°C (fluence Fⱼ < 1 J/cm²) to approximately 0.2/°C (fluences > 3 J/cm²).

The specifically tested fluence combinations are shown in Table 2 below. The actual realized temperatures Tₛ were measured by a thermal camera. Pain was evaluated using the standard VAS ("Visual Analogue Score") method.

**Table 2**

| **F₁ (J/cm²)** | **Fⱼ (J/cm²)** | **Fₛ (J/cm²)** |
|---|---|---|
| 5.5 | 0.0 | 5.5 |
| 5.5 | 0.4 | 5.9 |
| 4.8 | 1.0 | 5.8 |
| 3.6 | 2.2 | 5.8 |
| 2.8 | 3.4 | 6.2 |
| 2.6 | 4.6 | 7.2 |

In flash-lamp pumped laser, quickly altered pulse properties as outlined herein may be supplied by one and the same laser source (e.g., from the same laser oscillator), by quickly adjusting the current through the flashlamp. For example, when the current is determined by a voltage on a corresponding capacitor, and this voltage cannot be quickly changed, there is another option available. For lower level pumping and lower output energies, the pumping current pulse can for example be divided using an on and off switch into a series of shorter duration current pulses, effectively reducing the average (effective) current over the whole duration of the pumping.

Specific circuits for pumping an active medium of a laser that allow generating variously shaped pulses with different amplitudes even in quick succession with idle times in the range of just few ten microseconds or even less and that allows rendering the amplitude of the pulses independent of the storage capacitor(s) discharge rate are outlined in European patent application number EP 25152317.1 which was filed on January 16, 2025 and which is incorporated herein by reference in full.

## Claims

1. Apparatus for repetitively emitting trains of laser pulses (100) onto an organic tissue, each train of laser pulses including at least one laser pulse (110) of a first type and at least one laser pulse (120) of a second type;
wherein a time d between a last one of the at least one laser pulse of the first type and a first one of the at least one laser pulse of the second type is no more than 500 ms, preferably shorter than 300 ms, more preferably shorter than 100 ms;
wherein the at least one laser pulse of the first type comprises a fluence below an ablation threshold of the organic tissue, preferably at least 10% below the ablation threshold;
wherein the at least one laser pulse of the second type comprises a fluence above the ablation threshold of the organic tissue, preferably at least 10% above the ablation threshold; and
wherein the train of laser pulses starts with a pulse of the first type and/or ends with a pulse of the second type.

2. Apparatus according to claim 1, wherein the apparatus is configured such that a time between a last one of the at least one laser pulse of the second type of a train and a first one of the at least one laser pulse of the first type of the subsequent train is no more than 500 ms, preferably shorter than 300 ms, more preferably shorter than 100 ms.

3. Apparatus according to claim 1 or 2, wherein the at least one laser pulse of the first type forms a first group of subsequent laser pulses (1, 2, ... N₁), and/or wherein the at least one laser pulse of the second type forms a second group of subsequent laser pulses (1, 2, ..., N₂).

4. Apparatus according to one of the preceding claims, wherein the at least one laser pulse of the first type comprises more than 2 and less than 10 laser pulses of the first type, preferably with a repetition rate of 10 to 100 Hz, and the at least one laser pulse of the second type is a single laser pulse of the second type.

5. Apparatus according to one of the preceding claims, wherein the at least one laser pulse of the first type comprises a plurality of laser pulses of the first type, and the time d corresponds to an inverse of a repetition rate of the plurality of laser pulses of the first type.

6. Apparatus according to one of the preceding claims, wherein the apparatus is adapted to deliver at least one train of the laser pulses onto a same area of the organic tissue and/or wherein the apparatus is adapted to deliver pulses of a train of the laser pulses onto at least partially different areas of the organic tissue, preferably with a scanner.

7. Apparatus according to one of the preceding claims, wherein a cumulative fluence of the at least one laser pulse of the first type is less than 2.0 J/cm² and/or adapted such that the organic tissue is heated to a target temperature below a pain threshold temperature.

8. Apparatus according to one of the preceding claims, wherein the at least one laser pulse of the second type is adapted such that an ablation depth (530) of the at least one laser pulse in the organic tissue is below 100 µm, preferably below 50 µm, more preferably below 30 µm, most preferably below 10 µm.

9. Apparatus according to one of the preceding claims, wherein the at least one laser pulse of the second type comprises a fluence between 2.0 and 10 J/cm².

10. Apparatus according to one of the preceding claims, wherein a pulse length of the at least one laser pulse of the second type is shorter than a pulse length of the at least one laser pulse of the first type, preferably shorter than 400 µs, more preferably shorter than 330 µs, most preferably shorter than 250 µs.

11. Apparatus according to one of the preceding claims, wherein the at least one laser pulse of the first type and/or the at least one laser pulse of the second type is adapted such as to illuminate the area of the organic tissue with an illumination pattern having an effective surface coverage of more than 10% and less than 50 %.

12. Apparatus according to claim 11, wherein the illumination pattern comprises a honeycomb lattice, a ring lattice, and/or a polka dot lattice.

13. Apparatus according to one of the preceding claims, wherein the apparatus is adapted to selectively illuminate the organic tissue with two or more illumination patterns; and
wherein the apparatus is adapted to configure the time between the last laser pulse of the at least one laser pulse of the first type and the first laser pulse of the at least one laser pulse of the second type according to the selected illumination pattern.

14. Apparatus according to one of the preceding claims, wherein the apparatus comprises at least two predetermined modes selectable by the user, wherein at least one mode comprises emitting a train of laser pulses according to any one of the preceding claims.

15. Apparatus according to one of the preceding claims, wherein a wavelength of the at least one laser pulse of the second type and/or a wavelength of the at least one laser pulse of the first type comprises a water-absorbed wavelength, preferably an Er:YAG laser wavelength.

16. Method for a cosmetic treatment comprising:
emitting trains of laser pulses (100) onto an organic tissue, each train of laser pulses including at least one laser pulse (110) of a first type and at least one laser pulse (120) of a second type; wherein
a time d between a last one of the at least one laser pulse of the first type and a first one of the at least one laser pulse of the second type is no more than 500 ms, preferably shorter than 300 ms, more preferably shorter than 100 ms;
wherein the at least one laser pulse of the first type comprises a fluence below an ablation threshold of the organic tissue, preferably at least 10% below the ablation threshold;
wherein the at least one laser pulse of the second type comprises a fluence above the ablation threshold of the organic tissue, preferably at least 10% above the ablation threshold; and
wherein the train of laser pulses starts with a pulse of the first type and/or ends with a pulse of the second type.
